# EUROPEAN PATENT APPLICATION

(11) **EP 1 593 740 A1**
(43) Date of publication of application: **09.11.2005**
(21) Application number: 04745548.0
(22) Date of filing: 03.06.2004
(51) Int. Cl.: C12N 15/00, C12Q 1/68, A01K 67/027

(54) **METHOD OF SCREENING ANTI-OBESTIC MEDICINE, AND OBESITY MODEL ANIMAL**

(30) Priority: 06.06.2003 JP 2003163016; 05.04.2004 JP 2004111500
(71) Applicant: Astellas Pharma Inc., Tokyo 103-8411 (JP)
(72) Inventor: YASUNAGA, Kunio, Astellas Pharma Inc., Chuo-ku, Tokyo 103-8411 (JP); YAMAJI, Noboru, c/o Astellas Pharma Inc., Chuo-ku, Tokyo 103-8411 (JP); SUDA, Toshio, Chiyoda-ku, Tokyo 1020093 (JP); OIKE, Yuichi, Kumamoto-shi, Kumamoto 8620971 (JP)
(74) Representative: Bates, Philip Ian
(86) International application number: PCT/JP2004/007692
(87) International publication number: WO 2004/108920

(57) **Abstract**

A promoter for an angiopoietin-related growth factor (AGF), a vector comprising the promoter, a transformant comprising the promoter, and a method for screening an antiobesity agent, an antidiabetic agent, and/or a hypolipidemic agent by using the transformant, are disclosed. Further, an AGF knockout animal useful as an animal model for obesity, diabetes, and/or hyperlipemia is disclosed. Furthermore, an AGF transgenic animal useful for identification of a target molecule in a new drug development and/or a therapeutic agent for obesity, diabetes, and/or hyperlipemia is disclosed.

## Description

### TECHNICAL FIELD

The present invention relates to a method of screening antiobesity agents, an animal model of obesity, and a promoter for an angiopoietin-related growth factor (hereinafter referred to as AGF).

### BACKGROUND ART

Although the deleterious effect of obesity is widely known, there has been a remarkable increase in obesity in recent years. It is well-known that obesity (i.e., overaccumulation of fat in fatty tissues) causes various diseases, and thus it is proposed that obesity should be addressed as a disease to be treated. Diseases caused by obesity include, for example, lumbago, gonarthrosis, and osteoarthrosis. Such orthopedic diseases are directly caused by a gain in body weight due to obesity. The overaccumulation of fat associated with obesity causes diabetes, hyperlipemia, hypertension, or arteriosclerotic disease. In particular, it is known that an overaccumulation of visceral fat is involved in the development of such diseases (non-patent reference 1).

Basic methods for alleviating obesity include kinesitherapy and diet therapy, but to continue with such therapies is difficult. As methods other than the kinesitherapy and diet therapy, medicaments are used. At present, Sibutramine and orlistat are mainly used on a global scale. However, these medicaments have not only a weak, but also an adverse effect. In Japan, only mazindol is authorized, but the application thereof is limited to severe obesity, and the period of administration is also limited (non-patent reference 2).

Due to a modernization of society, the number of patients suffering from diabetes is rapidly increasing, not only in Japan but also globally. In particular, it is known that the development of type II diabetes having a number of patients is involved in obesity or overaccumulation of fat. As with obesity, treatments for type II diabetes include kinesitherapy and diet therapy, but medicaments are used because it is difficult to continue these therapies. Patients suffering from severe diabetes are treated with insulin, but the treatment with insulin has an adverse effect such as hypoglycemia. As oral hypoglycemic drugs, thiazolidinediones or sulfonylurea agents are mainly used. However, the thiazolidinediones have an adverse effect such as hepatopathy, edema, or heart failure, and the SU agents have an adverse effect such as the promotion of obesity, and thus, an agent for alleviating insulin resistance without an increase in body weight or such adverse effects is greatly desired (non-patent reference 3).

It is considered that adipocytokines capable of promoting insulin resistance are produced and secreted from hypertrophied adipocytes contained in the visceral fat of an obese patient suffering from diabetes, and act on adipocytes and/or myocytes close to the hypertrophied adipocytes to promote insulin resistance. In adipose tissues of patients suffering from diabetes, adipocytes become hypertrophied and change to tissues which are involved in the promotion of insulin resistance (non-patent references 4 and 5).

Leptin is well-known as a factor involved in the accumulation of adipose tissues which cause obesity or diabetes. Leptin is an inhibitory hormone for bodyweight gain, and it is known that a deficiency of leptin causes obesity by promoting the appetite and reducing energy consumption. The findings of such factors involved in the accumulation of adipose tissues and hypertrophy of adipocytes are very useful in developing therapeutic agents for diseases such as obesity, diabetes, or hyperlipemia (non-patent reference 6).

To identify a target molecule in a new drug development for obesity or diabetes, or to develop a therapeutic agent therefor, it is important to prepare transgenic mice in which genes involved in the diseases are modified, and to analyze the phenotypes thereof. A leptin-deficient mouse, an ob/ob mouse, becomes obese and shows insulin resistance, hyperinsulinemia, and a slight increase in blood glucose level. A leptin-receptor-deficient mouse, a db/db mouse, shows obesity, hyperinsulinemia, hyperleptinemia, hyperinsulinemia, and severe diabetes. These mice became obese and developed diabetes by the promotion of feeding and a reduced energy consumption, due to the deficiency of leptin action (non-patent reference 7). As a model mouse for obesity and type II diabetes, a KKA^{y} mouse is commonly used. The KKA^{y} mouse shows obesity and a reduction in insulin sensibility and develops hyperglycemia, and thus, is used in the research and development of a therapeutic agent for diabetes. However, no gene causing the above phenotypes of the KKA^{y} mouse has been identified (non-patent reference 8). It is considered that many unknown genes are involved in obesity or diabetes (non-patent reference 9). Therefore, it is desirable to find such genes, prepare transgenic mice, and analyze the phenotypes thereof, for developing medicaments for obesity or diabetes and elucidating the diseases.

An angiopoietin-related growth factor (AGF) is a secretory protein having a coiled-coil domain at the N-terminal side and a fibrinogen-like domain at the C-terminal side. The AGF is identical with NL8 reported in patent reference 1. It is reported that when CHO cells stably expressing NL8 are subcutaneously implanted into a nude mouse, the CHO cells exhibit tumorigenicity. Sequences identical or homologous with the AGF are disclosed in patent references 2 to 16. The references disclose an expression distribution thereof (patent references 4 and 5), an activity of inhibiting proliferation by stimulation of the vascular endothelial growth factor (VEGF) (patent reference 5), and a detection of overexpression thereof in human umbilical vein endothelial cell (HUVEC) (patent references 6 and 7), and further disclose that polypeptides consisting of amino acid sequences identical or homologous with the AGF are involved in angiogenesis, based on an expression in vascular tissues or the like, tumorigenicity, and/or homology with family molecules.
Non-patent reference 10 discloses that, in a transgenic mouse overexpressing the AGF in epidermal cells by using a K14 promoter, overangiogenesis occurred, microvessels under the skin were increased, and a proliferation of keratinocytes was activated. Non-patent reference 11 discloses that the AGF exhibits an activity of proliferating epidermal cells.

Because no AGF receptors were identified, tissues in which endogenous AGF functions and other physiological functions of AGF were unknown. Further, an antiobesity activity, an antidiabetic activity, or a hypolipidemic activity of AGF was unknown. Furthermore, an AGF promoter region has not been reported.
[non-patent reference 1] Metabolism, (U.S.A.), 1987, vol. 36, p.54-59
[non-patent reference 2] Nippon Rinsho, 2003, vol. 61, supplement 6, "Obesity", p.649-654
[non-patent reference 3] Nippon Rinsho, 2002, vol. 60, supplement 9, Shin-jidai no Tounyoubyougaku 3, p.310-331
[non-patent reference 4] Igaku no ayumi, 2000, vol. 192, p.513-518
[non-patent reference 5] Igaku no ayumi, 2000, vol. 192, p.541-545
[non-patent reference 6] Trends in Molecular Medicine, (Netherlands), 2002, vol. 8, no. 9, p.442-447
[non-patent reference 7] K. G. M. M. Alberti, Paul Zimmet, and R. A. DeFronzo ed., C. J. Bailey, INTERNATIONAL TEXTBOOK OF DIABETES MELLITUS, 2nd ed., (U.S.A.), John Wiley & Sons, Inc., 1997, p.23.1-23.25
[non-patent reference 8] Nippon Rinsho, 2002, vol. 60, supplement 18, p.38-44
[non-patent reference 9] Saishin Igaku, 2002, vol. 57, supplement March, p.536-544
[non-patent reference 10] Circulation, (U.S.A.), Nov. 5, 2002, vol. 106, no. 19, p.II-276-277
[non-patent reference 11] Two molecule of angiogenetic and anti-angiogenetic factor, -prevention of proliferation and metastasis of cancer-, THE NIKKAN KOGYO SHINBUN LTD., Oct. 2, 2002, p.5
[patent reference 1] International Publication No. WO99/15653
[patent reference 2] Japanese Unexamined Patent Publication (Kokai) No. 2000-300263
[patent reference 3] Japanese Translation Publication (Kohyo) No. 2001-517437
[patent reference 4] International Publication No. WO00/32221
[patent reference 5] International Publication No. WO00/53753
[patent reference 6] International Publication No. WO02/00690
[patent reference 7] International Publication No. WO02/08284
[patent reference 8] U.S. Patent Application Publication No. 2003/0105011
[patent reference 9] U.S. Patent Application Publication No. 2003/0105012
[patent reference 10] U.S. Patent Application Publication No. 2003/0105013
[patent reference 11] U.S. Patent No. 5,972,338
[patent reference 12] U.S. Patent No. 6,057,435
[patent reference 13] U.S. Patent No. 6,350,450
[patent reference 14] U.S. Patent No. 6,413,770
[patent reference 15] U.S. Patent No. 6,368,853
[patent reference 16] U.S. Patent No. 6,420,542

### DISCLOSURE OF INVENTION

### PROBLEMS TO BE SOLVED BY THE INVENTION

An object of the present invention is to provide a method of screening antiobestity agents and an animal model of obesity.

### MEANS FOR SOLVING THE PROBLEMS

The present inventors have conducted intensive studies, and, as a result, can now reveal that substances capable of promoting AGF have an antiobesity activity, an antidiabetic activity, and/or a hypolipidemic activity, by analyzing AGF knockout (KO) mice and AGF transgenic (Tg) mice. That is, the present inventors found that AGF knockout mice become remarkably obese, and that the AGF knockout mice are useful as an animal model of obesity (Example 4). Further, the present inventors found that, in the AGF knockout mice, there was an increase in weight of adipose tissues (Example 5), adipocytes were enlarged (Example 6), and there was an increase in each amount of triglyceride contained in skeletal muscles or liver (Example 7). In contrast, the present inventors found in the AGF transgenic mice (i.e., mice in which AGF was overexpressed) that an increase in body weight was suppressed (Example 4), that an increase in weight of adipose tissues was suppressed (Example 5), that an enlargement of adipocytes was suppressed (Example 6), and that each amount of triglyceride contained in skeletal muscles or liver was decreased (Example 7). Furthermore, the AGF knockout mice developed symptoms of diabetes in a glucose tolerance test (Example 8). From the above findings, the present inventors found that substances capable of promoting AGF have an antiobesity activity, an antidiabetic activity, and/or a hypolipidemic activity.

In addition, the present inventors obtained various lengths of upstream sequences of a human AGF gene, and have conducted intensive studies. As a result, the present inventors found that a short sequence of approximately 300 bp upstream of the AGF gene unexpectedly exhibited a promoter activity, whereas longer sequences of approximately 400 bp to approximately 3 kbp upstream of the AGF gene did not exhibit the promoter activity. Further, the present inventors established a method for screening a substance capable of promoting an AGF expression by utilizing a DNA having the above promoter activity. From the above findings, the present inventors found that the method for screening a substance capable of promoting an AGF expression is useful as a method for screening an antiobesity agent, an antidiabetic agent, and/or a hypolipidemic agent.
On the basis of the above findings, the present inventors provided an AGF knockout mouse, an AGF transgenic mouse, an AGF promoter, and a method for screening a substance capable of promoting AGF, and thus the present invention was completed.

The present invention relates to:
[1]
   (a) a DNA exhibiting a promoter activity for an angiopoietin-related growth factor, and consisting of a nucleotide sequence in which 1 to 10 nucleotides are substituted, deleted, added, and/or inserted in the nucleotide sequence consisting of nucleotides 2705-3001 of SEQ ID NO: 1, or
   (b) a DNA exhibiting a promoter activity for an angiopoietin-related growth factor, and consisting of a nucleotide sequence having a 90% or more homology with that consisting of nucleotides 2705-3001 of SEQ ID NO: 1,
[2] a DNA consisting of the nucleotide sequence consisting of nucleotides 2705-3001 of SEQ ID NO: 1,
[3] a recombinant vector characterized by comprising the DNA of [1] or [2] and exhibiting a promoter activity for an angiopoietin-related growth factor,
[4] a transformant characterized by comprising the DNA of [1] or [2] and exhibiting a promoter activity for an angiopoietin-related growth factor,
[5] a method for screening an antiobesity agent, an antidiabetic agent, and/or a hypolipidemic agent, characterized by comprising the steps of:
   i) bringing a substance to be tested into contact with the transformant of [4], and
   ii) measuring a promoter activity for an angiopoietin-related growth factor and analyzing a test substance dependent change in the promoter activity,
[6] the screening method of [5], wherein the transformant contains a reporter gene located downstream of the DNA of [1] or [2], and the promoter activity for an angiopoietin-related growth factor is measured by analyzing an expression of the reporter gene,
[7] a nonhuman knockout animal characterized in that a polynucleotide encoding an angiopoietin-related growth factor is functionally deficient on a chromosome, and
[8] a nonhuman transgenic animal which is a nonhuman animal or an offspring animal thereof obtained by ontogenesis from totipotent cells in which a polynucleotide is introduced together with a CAG promoter, wherein the polynucleotide is carried on a chromosome, a polypeptide encoded by the polynucleotide is expressed in a somatic cell, and the polypeptide is selected from the group consisting of:
   (a) a polypeptide exhibiting an activity of suppressing an increase in body weight, and comprising an amino acid sequence consisting of amino acids 1-450 of SEQ ID NO: 3 or amino acids 1-433 of SEQ ID NO: 5,
   (b) a polypeptide exhibiting an activity of suppressing an increase in body weight, and comprising an amino acid sequence in which 1 to 10 amino acids are substituted, deleted, and/or inserted in an amino acid sequence consisting of amino acids 1-450 of SEQ ID NO: 3 or amino acids 1-433 of SEQ ID NO: 5,
   (c) a polypeptide exhibiting an activity of suppressing an increase in body weight, and encoded by a DNA which hybridizes under stringent conditions to a DNA encoding an amino acid sequence consisting of amino acids 1-450 of SEQ ID NO: 3 or amino acids 1-433 of SEQ ID NO: 5, and
   (d) a polypeptide exhibiting an activity of suppressing an increase in body weight, and comprising an amino acid sequence having a 95% or more homology with that consisting of amino acids 1-450 of SEQ ID NO: 3 or amino acids 1-433 of SEQ ID NO: 5.

Further, the present invention includes a method for preparing a nonhuman transgenic animal (preferably a mouse) comprising the steps of:
introducing a DNA construct in which a CAG promoter is linked to the upstream of an AGF cDNA, into an embryonic stem (ES) cell,
selecting a clone in which the AGF is introduced,
introducing the selected clone into blastocysts,
transferring the manipulated eggs to a uterus of a pseudopregnant female nonhuman mammal (preferably a mouse), and
breeding the nonhuman mammals (preferably mouse) and
selecting, from the offspring thereof, an animal having the DNA encoding the AGF on the genome.
Furthermore, the present invention includes a method for preparing a nonhuman knockout animal (preferably a mouse) comprising the steps of:
introducing a DNA construct in which a part of a genomic sequence containing ORF (Open Reading Frame) of the AGF gene is replaced with a drug resistant gene (such as a neomycin resistant gene), into an ES cell,
culturing the ES cell in the presence of an appropriate drug (such as G418) to select a resistant strain,
introducing the resistant strain into blastocysts,
transferring the manipulated eggs to a uterus of a pseudopregnant female nonhuman mammal (preferably a mouse), to obtain chimeric animals,
mating each chimeric animal (preferably a mouse) with a normal animal (preferably a mouse) to obtain heterozygous animals (preferably mice), and
mating the heterozygous animals with each other to obtain homozygous animals (preferably mice).

The term "transgenic animal" as used herein means an animal in which a promoter and a gene are introduced into a chromosome to overexpress the gene at a desired location. The term "knockout animal" as used herein means an animal in which an expression of a particular gene is deleted by gene manipulation of a chromosome.

The patent references 1 to 16 disclose polypeptides which may be used in preparing the transgenic animal of the present invention and are identical or homologous with a human or mouse AGF consisting of the amino acid sequence of SEQ ID NO: 3 or 5. These patent references disclose that the polypeptides identical or homologous with the human or mouse AGF consisting of the amino acid sequence of SEQ ID NO: 3 or 5 are involved in angiogenesis, based on expressions in vascular tissues or the like, tumorigenicity, and/or homologies with family molecules. The patent references 4 to 9 disclose many diseases including diabetes, but disclose no support or evidence. Further, the non-patent reference 10 discloses a functional analysis of transgenic mice in which a K14 promoter was used to overexpress AGF in epidermal cells, and the non-patent reference 11 discloses that AGF exhibits an epidermal cell proliferating activity. WO03/083114 (patent reference 17), published after the priority date of the present application, and Proceedings of the National Academy of Sciences of the United States of America, (U.S.A.), 2003, Vol. 100, p. 9494-9499 (non-patent reference 12) disclose that transgenic mice in which AGF was overexpressed in epidermal cells utilizing a K14 promoter were used to find that AGF exhibits an angiogenetic activity, an epidermal cell proliferating activity, a chondrocyte proliferating activity, an activity of promoting wound healing, and a tissue generative activity. However, because no AGF receptors were identified, tissues in which endogenous AGF functions and other physiological functions of AGF were unknown. Further, an antiobesity activity or a hypolipidemic activity of AGF was unknown.

A sequence of 129048 bp containing the sequence consisting of nucleotides 2705-3001 of SEQ ID NO: 1 is registered as accession No. AC020931 in the Genbank database. However, neither the use nor activities (including an AGF promoter activity) of the sequence are disclosed in the database.

Although transgenic mice in which a K14 promoter was used for overexpression in epidermal cells were reported (non-patent reference 10, non-patent reference 12, and patent reference 17), there were no reports in which a transgenic mouse systemically expressing AGF and an AGF knockout mouse were generated and analyzed.

The present inventors first generated AGF transgenic mice systemically overexpressing AGF and AGF knockout mice, and unexpectedly found that the AGF knockout mice are useful as an animal model for obesity, diabetes, and/or hyperlipemia, and that the AGF transgenic mice systemically overexpressing AGF are useful for identification of a target molecule in a new drug development and a therapeutic agent for obesity, diabetes, and/or hyperlipemia.

Further, the present inventors first revealed that AGF or substances capable of promoting AGF have an antiobesity activity, an antidiabetic activity, and/or a hypolipidemic activity, by analyzing the AGF knockout mice and the AGF transgenic mice systemically overexpressing AGF. The present inventors first obtained an upstream sequence of the AGF gene having an AGF promoter activity, and established a method for screening a substance capable of promoting an AGF expression by utilizing the above sequence. That is, the AGF knockout nonhuman animal, the AGF transgenic nonhuman animal, the AGF promoter, and the method for screening a substance capable of promoting AGF (i.e., an antiobesity agent, an antidiabetic agent, and/or a hypolipidemic agent) were first provided by the present inventors.

### EFFECTS OF THE INVENTION

According to the AGF promoter of the present invention, a screening system for an antiobesity agent, an antidiabetic agent, and/or a hypolipidemic agent can be provided.
A substance which is obtained by the screening method of the present invention and is capable of promoting an AGF expression is useful as an active ingredient of an antiobesity agent, an antidiabetic agent, and/or a hypolipidemic agent.
The nonhuman AGF knockout animal of the present invention is useful as an animal model for obesity, diabetes, and/or hyperlipemia.
The nonhuman transgenic animal of the present invention may be used for identification of a target molecule in a new drug development and/or a therapeutic agent for obesity, diabetes, and/or hyperlipemia.

### BEST MODE FOR CARRYING OUT THE INVENTION

### [1] DNA, recombinant vector, and transformant of the present invention

The present inventors used a human genomic library to obtain partial sequences of approximately 200 bp, 300 bp, 400 bp, 600 bp, 800 bp, 1 kbp, 1.3 kbp, and 3 kbp located upstream of the human AGF gene, and determined the nucleotide sequences thereof (nucleotide sequences consisting of nucleotides 2790-3001, nucleotides 2705-3001, nucleotides 2604-3001, nucleotides 2406-3001, nucleotides 2206-3001, nucleotides 2021-3001, nucleotides 1640-3001, and nucleotides 1-3001 of SEQ ID NO: 1). Each DNA fragment was subcloned into plasmid pGV-B2 containing a luciferase gene as a reporter gene. It was confirmed whether the obtained upstream DNA regions exhibit a promoter activity by detecting an expression of the reporter gene in each fused plasmid (i.e., luciferase activity). As a result, although the DNAs of approximately 400 bp to 3 kbp did not exhibit the promoter activity, the shorter DNA of approximately 300 bp unexpectedly exhibited the promoter activity. In this connection, the further shorter DNA of approximately 200 bp did not exhibit the promoter activity. The DNA of approximately 300 bp can be used to screen a substance capable of regulating the AGF promoter activity, i.e., an antiobesity agent, an antidiabetic agent, and/or a hypolipidemic agent.

The DNA of the present invention includes, for example,
1) a DNA consisting of the nucleotide sequence consisting of nucleotides 2705-3001 of SEQ ID NO: 1,
2) a DNA exhibiting a promoter activity for an angiopoietin-related growth factor, and consisting of a nucleotide sequence in which 1 to 10 (preferably 1 to 7, more preferably 1 to 5, most preferably 1 to 3) nucleotides are substituted, deleted, added, and/or inserted in the nucleotide sequence consisting of nucleotides 2705-3001 of SEQ ID NO: 1, and
3) a DNA exhibiting a promoter activity for an angiopoietin-related growth factor, and consisting of a nucleotide sequence having a 90% or more (preferably 95% or more, more preferably 97% or more) homology with that consisting of nucleotides 2705-3001 of SEQ ID NO: 1.

The term "promoter activity for an angiopoietin-related growth factor (AGF)" as used herein means a promoter activity in the AGF gene, particularly a promoter activity of the DNA consisting of the nucleotide sequence consisting of nucleotides 2705-3001 of SEQ ID NO: 1. A method for judging whether or not a DNA of interest exhibits the "AGF promoter activity" is not limited, but the promoter activity may be confirmed by a known conventional method. For example, an appropriate reporter gene DNA is linked at the 3' downstream of a DNA to be judged, and the DNA construct is introduced into eukaryotic cells (preferably animal cell line). The cells are cultured, and an amount of the reporter gene expressed in the cells is measured to confirm the AGF promoter activity. More particularly, the AGF promoter activity may be confirmed in accordance with the method described in Example 12.

The DNA of the present invention may be prepared by, but is not limited to, the following methods.

### (1) Preparation using PCR method

A DNA consisting of the nucleotide sequence consisting of nucleotides 2705-3001 of SEQ ID NO: 1 may be prepared by synthesizing a primer set of a primer consisting of a nucleotide sequence located at the 5' side of the sequence consisting of nucleotides 2705-3001 of SEQ ID NO: 1 and a primer consisting of a nucleotide sequence complementary to a sequence located at the 3' side thereof (preferably a primer set of a primer consisting of the nucleotide sequence of SEQ ID NO: 47 and a primer consisting of the nucleotide sequence of SEQ ID NO: 46), and performing a PCR using these primers and a human genomic DNA. The human genomic DNA may be prepared from an appropriate human tissue in accordance with a conventional method. A commercially available human genomic DNA may be used. More particularly, the DNA of the present invention may be prepared in accordance with, but is not limited to, the method described in Example 11. Instead of the primers used in the method described in Example 11, other primers having restriction enzyme recognition sites different from those contained in the nucleotide sequences of SEQ ID NOS: 47 and 46, or other primers having lengths different from those of the nucleotide sequences of SEQ ID NOS: 47 and 46, may be used.

### (2) Preparation using DNA synthesis

The DNA of the present invention may be prepared by chemically synthesizing the nucleotide sequence consisting of nucleotides 2705-3001 of SEQ ID NO: 1 and the complementary strand, as several partial DNA fragments, and ligating these fragments with each other. The DNA fragments may be synthesized using a DNA synthesizer [for example, Oligo 1000M DNA Synthesizer (Beckman) or 394 DNA/RNA Synthesizer (Applied Biosystems)].

Those skilled in the art may prepare a DNA having the promoter activity similar to that of a naturally occurring promoter DNA, by performing a modification (for example, substitution, deletion, and/or addition) of the nucleotide sequence of the naturally occurring promoter DNA. The DNA of the present invention includes such a DNA having a nucleotide sequence in which one or more nucleotides are substituted, deleted, added, and/or inserted in the nucleotide sequence of a naturally occurring promoter DNA and exhibiting the promoter activity similar to that of the naturally occurring promoter DNA. The nucleotide modification may be carried out in accordance with, for example, a method for introducing deletion by a restriction enzyme or DNA exonuclease, a method for introducing mutation by a site-directed mutagenesis [Nucleic Acid Res. 10, 6487 (1982)], a method for modifying a promoter sequence by a PCR method using a mutated primer, or a method for directly introducing a synthetic mutated DNA [Maniatis, T. et al. (1989): "Molecular Cloning - A Laboratory Manual 2^{nd} Edit." Cold Spring Harbor Laboratory, NY].

Whether or not the obtained DNA exhibits the promoter activity may be confirmed by a known conventional method. For example, an appropriate reporter gene DNA is linked at the 3' downstream of the obtained DNA, and the DNA construct is introduced into eukaryotic cells (preferably animal cell line). The cells are cultured, and an amount of the reporter gene expressed in the cells is measured to confirm the promoter activity. More particularly, the promoter activity may be confirmed in accordance with the method described in Example 12.

The recombinant vector of the present invention may be prepared by integrating the DNA of the present invention into a vector appropriately selected in accordance with a desired purpose. In the vector, a DNA containing a structural gene to be expressed may be inserted at the 3' downstream of the DNA of the present invention. The structural gene is not particularly limited, so long as it encodes a protein. As the structural gene, the whole or part of ORF (Open Reading Frame) may be used. For example, as described in Example 11, the recombinant vector of the present invention may be preferably prepared by integrating the DNA of the present invention (i.e., DNA having the AGF promoter activity) into a vector containing a reporter gene such as luciferase. The "reporter gene" is not particularly limited, so long as it is commonly used. As the reporter gene, an enzyme gene in which a quantitative measurement may be easily performed is preferable. As the enzyme gene, there may be mentioned, for example, a chloramphenicol acetyl transferase gene (CAT) derived from a bacterial transposon, a luciferase gene (Luc) derived from a firefly, or a green fluorescent protein gene (GFP) derived from a jellyfish. The recombinant vector of the present invention may be preferably prepared in accordance with the method described in Example 11. Whether or not the obtained recombinant vector exhibits the AGF promoter activity may be confirmed by a known conventional method. For example, the recombinant vector is introduced into eukaryotic cells (preferably animal cell line), the cells are cultured, and an amount of the reporter gene expressed in the cells is measured to confirm the promoter activity. More particularly, the promoter activity may be confirmed in accordance with the method described in Example 12.

The transformant of the present invention may be prepared by introducing the recombinant vector containing the DNA of the present invention into a host cell appropriately selected in accordance with a desired purpose. For example, to construct a screening system for a substance capable of regulating the hAGF promoter activity, a cell derived from mammals such as a human, a mouse, or a rat (preferably a human-derived cell) may be used. As such a cell line, a cell having transcriptional regulatory factors or the like located in a commonly used cell may be used, and there may be mentioned, for example, 293EBNA, HT-1080, or HepG2, which are commercially available.

As a method for introducing a vector into a host cell, there may be mentioned, for example, a DEAE-dextran method [Luthman, H. and Magnusson, G. (1983) Nucleic Acids Res., 11, 1295-1308], a calcium phosphate-DNA coprecipitation method [Graham, F. L. and van der Ed, A. J. (1973) Virology, 52, 456-457], a method using FuGENE6 (Nippon Roche), or an electroporation method [Neumann, E. et al.(1982) EMBO J., 1, 841-845].

Whether or not the obtained transformant exhibits the AGF promoter activity may be confirmed by measuring an amount of the reporter gene expressed in the transformant. More particularly, it may be confirmed in accordance with the method described in Example 12. A transformant having the AGF promoter activity may be selected by comparing it with that transfected with a recombinant vector without the DNA of the present invention.

### [2] Screening method of the present invention

As shown in Examples 4 to 10, AGF exhibits an antiobesity activity, an antidiabetic activity, and/or a hypolipidemic activity. Therefore, a substance useful as an antiobesity agent, an antidiabetic agent, and/or a hypolipidemic agent may be obtained by screening a substance capable of promoting the AGF promoter activity.

More particularly, an antiobesity agent, an antidiabetic agent, and/or a hypolipidemic agent may be screened by the screening method of the present invention, characterized by comprising the steps of:
i) bringing a substance to be tested into contact with the transformant of the present invention, and
ii) measuring the hAGF promoter activity, and analyzing (for example, detecting or measuring) a test substance dependent change in the promoter activity.
Preferably, an antiobesity agent, an antidiabetic agent, and/or a hypolipidemic agent may be screened by the screening method of the present invention, characterized by comprising the steps of:
i) bringing a substance to be tested into contact with a cell transfected with a reporter gene fused to the DNA (preferably an hAGF promoter region consisting of the nucleotide sequence consisting of nucleotides 2705-3001 of SEQ ID NO: 1) of the present invention, and
ii) analyzing a test substance dependent change in the reporter activity, in accordance with an expression of the reporter gene as an index.

A reporter gene assay (Tamura et al., "Tensha inshi kenkyuhou", YODOSHA, 1993) is a method in which regulation of a gene expression is analyzed (for example, detected or measured) on the basis of an expression of a reporter gene as a marker. A gene expression is generally regulated by a promoter region located at the 5' upstream of the gene, and thus an amount of the gene expressed at the transcriptional stage may be estimated by measuring the promoter activity. When a test substance activates the promoter, the transcription of a reporter gene located downstream of the promoter region is activated. That is, the action of activating a promoter (i.e., the action of promoting an expression) may be detected by replacing it with an expression of the reporter gene. Therefore, the action of a test substance on the regulation of AGF expression may be detected by the reporter gene assay using the AGF promoter region, on the basis of the expression of the reporter gene. The "reporter gene" fused to the DNA (preferably an hAGF promoter region consisting of the nucleotide sequence consisting of nucleotides 2705-3001 of SEQ ID NO: 1) of the present invention is not particularly limited, so long as it is commonly used. As the reporter gene, an enzyme gene in which a quantitative measurement may be easily performed is preferable. As the enzyme gene, there may be mentioned, for example, a chloramphenicol acetyl transferase gene (CAT) derived from a bacterial transposon, a luciferase gene (Luc) derived from a firefly, or a green fluorescent protein gene (GFP) derived from a jellyfish. The reporter gene may be functionally fused to the DNA (preferably an hAGF promoter region consisting of the nucleotide sequence consisting of nucleotides 2705-3001 of SEQ ID NO: 1) of the present invention. The reporter gene fused to the DNA (preferably the hAGF promoter region) of the present invention is stably or transiently expressed in cells such as animal cells or yeast. An amount of the reporter gene expressed in the transformants when a test substance is brought into contact therewith may be compared to that when a test substance is not added, and a test substance dependent change in the promoter activity may be analyzed.

The transformant may be prepared in accordance with the methods described in the above [1]. A method for analyzing an amount of a reporter gene expressed may be appropriately selected in accordance with a protein encoded by the reporter gene. For example, when the reporter gene encodes a fluorescent protein such as luciferase, an amount of the reporter gene expressed may be determined by dissolving the transformants by an appropriate method to obtain a cell lysate, adding luciferin as a substrate to a supernatant of the cell lysate, and measuring fluorescence by an appropriate fluorescence detector (for example, ML3000; Dinatech laboratories). The reaction may be carried out using a commercially available detection kit, for example, Luciferase Assay System (Promega).

A substance capable of promoting the AGF expression, i.e., an antiobesity agent, an antidiabetic agent, and/or a hypolipidemic agent, may be screened by carrying out the above steps. More particularly, the method described in Example 12 is preferable as the screening method. As the substance capable of promoting the promoter activity, it is preferable to select a substance in which the activity of activating the promoter is 1.5 times or more with respect to that in the absence of the substance.

Substances to be tested which may be used in the screening method of the present invention are not particularly limited, but there may be mentioned, for example, commercially available compounds (including peptides), various known compounds (including peptides) registered in chemical files, compounds obtained by combinatorial chemistry techniques (Terrett et al., J. Steele. Tetrahedron, 51, 8135-8137, 1995), culture supernatants of microorganisms, natural components derived from plants or marine organisms, animal tissue extracts, or compounds (including peptides) obtained by chemically or biologically modifying compounds (including peptides) selected by the screening method of the present invention.

### [3] The nonhuman knockout animal and nonhuman transgenic animal of the present invention

The nonhuman knockout animal of the present invention is not particularly limited, so long as a polynucleotide encoding AGF is functionally deficient on a chromosome. The nonhuman knockout animal may be prepared in accordance with a method commonly used in preparing a knockout animal [for example, see "Saisin doubutsu saibou jikken manual", published by LIC, Chapter 7, p. 361-408 (1990)], by utilizing a genomic sequence containing ORF of the AGF gene or a genomic sequence containing the upstream and/or downstream sequences of the AGF gene. The genomic sequence may be selected in accordance with an animal (species) to be used. For example, when a mouse is used, the sequence of accession No. AC073775.2 in GenBank may be utilized. Alternatively, the AGF knockout animal may be obtained by selecting a mouse functionally deficient in the AGF gene from mice prepared by a random mutagenesis method (for example, see Nature, 392, 608-611, 1998).

More particularly, the nonhuman knockout animal of the present invention may be prepared in accordance with, for example, the method described in Example 1. That is, a genomic sequence containing ORF of the AGF gene is used to prepare a DNA construct in which a part thereof is replaced with a drug resistant gene (such as a neomycin resistant gene). ES cells are transfected with the DNA construct, and cultured in the presence of an appropriate drug (such as G418) to obtain resistant strains. The resistant strains are analyzed by, for example, Southern blotting, to select clones in which a desired homologous recombination occurs. Each clone is microinjected into a blastocyst, and the manipulated eggs are transferred to a uterus, to obtain chimeric mice. Each chimeric mouse is mated with a normal mouse to obtain heterozygous mice. Further, the heterozygous mice are mated with each other to obtain homozygous mice in accordance with the Mendel's laws.

Methods not utilizing ES cells, such as a method in which a mixture containing a gene of interest and eggs is treated with calcium phosphate, a method in which a gene is directly introduced into a nucleus in fertilized eggs at the pronuclear stage under a phase-contrast microscope (a microinjection method; U.S. patent No. 4,873,191), a method in which eggs are infected with a retroviral vector containing a gene, or a sperm vector method in which a gene is introduced into eggs via sperm [M.Lavitrano et al., Cell 57(5): 717-723 (1989)], are known, and may be used in preparing the nonhuman knockout animal (or nonhuman transgenic animal described below) of the present invention.

The nonhuman knockout animal (or nonhuman transgenic animal described below) of the present invention may be prepared by utilizing any vertebrates other than a human. Various vertebrates, such as a mouse, rat, rabbit, miniature pig, goat, sheep, or cattle, were used to generate knockout animals in which various genes were incorporated or an expression level was modified. Such species are included in the nonhuman knockout animal of the present invention. As the nonhuman knockout animal (or nonhuman transgenic animal described below) of the present invention, rodents are preferable, and a mouse is most preferable.

As described in Examples, the nonhuman knockout animal of the present invention becomes remarkably obese (Example 4). Further, in the nonhuman knockout animal of the present invention, there is an increase in weight of adipose tissues (Example 5), adipocytes are enlarged (Example 6), there is an increase in each amount of triglyceride contained in skeletal muscles or liver (Example 7), and symptoms of diabetes are developed in a glucose tolerance test (Example 8). These phenotypes resemble symptoms in obese subjects or patients suffering from diabetes or hyperlipemia, and thus the nonhuman knockout animal of the present invention is useful as an animal model for obesity, diabetes, and/or hyperlipemia. That is, the nonhuman knockout animal of the present invention can be used not only in screening medicaments for treating or preventing obesity, diabetes, and/or hyperlipemia, but also in elucidating the mechanisms of such diseases and for a safety test of the medicament screened. The present invention includes the use of the nonhuman AGF knockout animal as an animal model for obesity, diabetes, and/or hyperlipemia.

The nonhuman transgenic animal of the present invention may be prepared in accordance with the above procedures described with respect to the nonhuman knockout animal of the present invention, except that a polynucleotide which encodes a polypeptide (hereinafter referred to as "polypeptide for preparing a transgenic animal") selected from the group consisting of:
(a) a polypeptide exhibiting an activity of suppressing an increase in body weight, and comprising an amino acid sequence consisting of amino acids 1-450 of SEQ ID NO: 3 or amino acids 1-433 of SEQ ID NO: 5,
(b) a polypeptide exhibiting an activity of suppressing an increase in body weight, and comprising an amino acid sequence in which 1 to 10 amino acids are substituted, deleted, and/or inserted in an amino acid sequence consisting of amino acids 1-450 of SEQ ID NO: 3 or amino acids 1-433 of SEQ ID NO: 5,
(c) a polypeptide exhibiting an activity of suppressing an increase in body weight, and encoded by a DNA which hybridizes under stringent conditions to a DNA encoding an amino acid sequence consisting of amino acids 1-450 of SEQ ID NO: 3 or amino acids 1-433 of SEQ ID NO: 5, and
(d) a polypeptide exhibiting an activity of suppressing an increase in body weight, and comprising an amino acid sequence having a 95% or more homology with that consisting of amino acids 1-450 of SEQ ID NO: 3 or amino acids 1-433 of SEQ ID NO: 5
and is linked to a CAG (modified chicken beta-actin promoter with CMV-IE enhancer) promoter [GENE, 108(1991) 193-200] is used as a gene to be introduced.

As the polypeptide for preparing a transgenic animal, a human AGF consisting of the amino acid sequence consisting of amino acids 1-450 of SEQ ID NO: 3, or a mouse AGF consisting of the amino acid sequence consisting of amino acids 1-433 of SEQ ID NO: 5 is preferable.

The amino acid sequence of SEQ ID NO: 3 is that of a human AGF precursor. The human AGF has a signal sequence (-20 to -1) at the N terminus thereof, and the signal sequence is cleaved when the precursor is secreted to the outside of cells. A human matured AGF, which is generated by cleaving the signal sequence and is composed of the amino acid sequence consisting of amino acids 1-450 of SEQ ID NO: 3, has physiological activities.
Similarly, the amino acid sequence of SEQ ID NO: 5 is that of a mouse AGF precursor. The mouse AGF has a signal sequence (-24 to -1) at the N terminus thereof, and the signal sequence is cleaved when the precursor is secreted to the outside of cells. A mouse matured AGF, which is generated by cleaving the signal sequence and is composed of the amino acid sequence consisting of amino acids 1-433 of SEQ ID NO: 5, has physiological activities.

As the above polypeptide (b) which may be used as the polypeptide for preparing a transgenic animal [i.e., a polypeptide exhibiting an activity of suppressing an increase in body weight, and comprising an amino acid sequence in which 1 to 10 (for example, one to several) amino acids are substituted, deleted, and/or inserted in an amino acid sequence consisting of amino acids 1-450 of SEQ ID NO: 3 or amino acids 1-433 of SEQ ID NO: 5], there may be mentioned, for example, a polypeptide exhibiting an activity of suppressing an increase in body weight, and comprising an amino acid sequence in which preferably 1 to 7, more preferably 1 to 5 amino acids are substituted, deleted, and/or inserted in an amino acid sequence consisting of amino acids 1-450 of SEQ ID NO: 3 or amino acids 1-433 of SEQ ID NO: 5.

To maintain the functions of the original polypeptide, the amino acid to be substituted is preferably an amino acid having properties similar to those of the original amino acid. For example, amino acids belonging to each of the following groups have properties similar to those of other members in the group. When these amino acids are substituted with other amino acids in the same group, the essential functions of the original protein are often maintained. Such amino acid substitution is called a conservative substitution, and is known as a method for changing an amino acid sequence while maintaining the polypeptide functions.
Nonpolar amino acids: Ala, Val, Leu, Ile, Pro, Met, Phe, and Trp
Uncharged amino acids: Gly, Ser, Thr, Cys, Tyr, Asn, and Gln Acidic amino acids : Asp and Glu
Basic amino acids: Lys, Arg, and His

A method for judging whether or not a polypeptide of interest exhibits the activity of suppressing an increase in body weight is not limited, but it may be confirmed by, for example, the method described in Example 4. That is, the activity may be confirmed by breeding transgenic animals, prepared by utilizing a gene encoding the polypeptide, with a normal diet or a high fat diet, and comparing changes in body weight with those of wild-type animals.

With respect to the above polypeptide (c) which may be used as the polypeptide for preparing a transgenic animal, the "stringent conditions" include, as hybridization conditions, conditions of "5 x SSPE, 5 x Denhard's solution, 0.5% sodium dodecyl sulfate (SDS), 40% formamide, and 200 µg/mL salmon sperm DNA, at 37°C overnight", and, as more stringent hybridization conditions, conditions of "5 x SSPE, 5 x Denhard's solution, 0.5% SDS, 50% formamide, and 200 µg/mL salmon sperm DNA, at 42°C overnight". Further, washing conditions include mild conditions such as "5 x SSC and 1% SDS, at 42°C", usual conditions such as "0.5 x SSC and 0.1% SDS, at 42°C", and more stringent conditions such as "0.2 x SSC and 0.1% SDS, at 65°C". The "5 x SSPE" contains 50 mmol/L sodium phosphate (pH 7.4), 0.75 mol/L NaCl, and 5 mmol/L EDTA. The "5 x SSC" contains 0.75 mol/L NaCl and 75 mmol/L sodium citrate (pH 7.0).

The homology in the above polypeptide (d) which may be used as the polypeptide for preparing a transgenic animal is at least 95% or more, preferably 97% or more. The homology between amino acid sequences may be calculated by a BLAST search algorithm. More particularly, it may be calculated using a b12seq program (Tatiana A. Tatusova and Thomas L. Madden, FEMS Microbiol. Lett., 174, 247-250, 1999) in a BLAST package (sgi32bit edition, version 2.0.12; obtained from NCBI) in accordance with a default parameter. As a pairwise alignment parameter, a program "blastp" is used. Further, "0" as a Gap insertion cost value, "0" as a Gap elongation cost value, "SEG" as a filter for a Query sequence, and "BLOSUM62" as a Matrix are used, respectively.

As the polypeptide for preparing a transgenic animal, a polypeptide in which a signal sequence is added to the N terminus of any one of the polypeptides (a) to (d) is preferable. The signal sequence is not particularly limited, so long as it may lead a polypeptide to pass through the membrane. As the signal sequence, signal sequences described in Biochemistry, 28(3), 923-930, 1989 may be used. As the polypeptide for preparing a transgenic animal, a polypeptide in which a signal sequence (-20 to -1) in the amino acid sequence of SEQ ID NO: 3 or a signal sequence (-24 to -1) in the amino acid sequence of SEQ ID NO: 5 is added to the N terminus of any one of the polypeptides (a) to (d) is more preferable, and a polypeptide consisting of the amino acid sequence of SEQ ID NO: 3 or 5 is most preferable.

The origin of the polypeptide for preparing a transgenic animal is not limited to a human or a mouse. For example, a polypeptide derived from organisms other than a human or a mouse, or a polypeptide obtained, using genetic engineering techniques, by artificially modifying an amino acid sequence consisting of amino acids 1-450 of SEQ ID NO: 3 or amino acids 1-433 of SEQ ID NO: 5, may be used, so long as it is included in any one of the polypeptides (a) to (d).

The polynucleotides encoding the polypeptides (a) to (d), i.e., the polynucleotides which may be used in preparing the nonhuman transgenic animal of the present invention, include DNAs and RNAs, and DNAs are preferable. As the polynucleotide, there may be mentioned, for example, a polynucleotide which encodes a polypeptide having an activity of suppressing an increase in body weight and contains a nucleotide sequence consisting of nucleotides 61-1410 of SEQ ID NO: 2 or nucleotides 73-1371 of SEQ ID NO: 4. A polynucleotide consisting of a nucleotide sequence of SEQ ID NO: 2 or 4, or a polynucleotide consisting of a nucleotide sequence consisting of nucleotides 61-1410 of SEQ ID NO: 2 or nucleotides 73-1371 of SEQ ID NO: 4, is preferable.

The nonhuman transgenic animal of the present invention may be prepared, for example, by the method described in Example 2. That is, a CAG promoter is linked to the upstream of an AGF cDNA, to prepare a DNA construct. The obtained DNA construct is introduced into ES cells, and clones expressing AGF are selected by, for example, a Western blotting analysis. Each clone is microinjected into a blastocyst, and the manipulated eggs are transferred to a uterus, to obtain chimeric mice. Each chimeric mouse is mated with a normal mouse to obtain transgenic mice.

The nonhuman transgenic animal of the present invention can be used for identification of a target molecule in a new drug development and/or a therapeutic agent for obesity, diabetes, and/or hyperlipemia. For example, the nonhuman transgenic animal of the present invention (such as an AGF transgenic mouse) is mated with an animal (such as a mouse) in which a gene X of interest is modified, to obtain offspring in which both genes are modified. The phenotypes of the offspring may be analyzed. When the offspring in which both genes are modified do not exhibit an antiobesity activity, an antidiabetic activity, and/or a hypolipidemic activity, it is revealed that the gene X has an antiobesity activity, an antidiabetic activity, and/or a hypolipidemic activity. Further, it is revealed that the gene X and/or an agent for promoting the function of the gene X may be used as a therapeutic agent for obesity, diabetes, and/or hyperlipemia.

Further, a gene whose expression is changed in the nonhuman transgenic animal of the present invention is considered to be a target molecule in a new drug development for obesity, diabetes, and/or hyperlipemia, and thus the gene, or agonists or antagonists thereof may be used as an antiobesity agent, an antidiabetic agent, and/or a hypolipidemic agent. The nonhuman transgenic animal of the present invention is useful in providing materials (such as tissues or blood) for identification of a target molecule in a new drug development.

### [EXAMPLES]

The present invention now will be further illustrated by, but is by no means limited to, the following Examples. In this connection, the following procedures may be performed in accordance with known methods (for example, "Molecular Cloning", Sambrook, J. et al., Cold Spring Harbor Laboratory Press, 1989), unless otherwise specified. Further, when a commercially available reagent or kit is used, procedures may be performed in accordance with a protocol attached thereto.

Knockout animals and transgenic animals may be prepared in accordance with "Manipulating the Mouse Embryo. A Laboratory Manual." 2nd Edition, B. Hogan, R. Beddington, F. Costantini, E. Lacy, Cold Spring Harbor, New York, Cold Spring Harbor Laboratory Press, 1994, unless otherwise specified. Further, chimeric mice may be prepared by using ES cells in accordance with AL Joyner: "Gene Targeting, A Practical Approach", OXFORD UNIVERSITY PRESS, 1993; or Shinich Aizawa, Biomanual series 8, "Gene Targeting, ES saibou wo mochiita henimausu no sakusei", Youdosha, 1994, unless otherwise specified.

### Example 1: Preparation of AGF KO mice

### (1) construction of targeting vector

A targeting vector containing a genomic sequence (5' long arm) at the 5' side of the mouse AGF gene, a pgk promoter, a neomycin resistant gene, a genomic sequence (3' short arm) containing a part of exon 2 and the whole of exon 3 in the mouse AGF gene, and an HSV-tk gene, in this order, was prepared in accordance with the following procedures.

A cDNA corresponding to the full-length of the coding region of mouse AGF protein was prepared by the procedures described in Example 1 of WO03/083114. The cDNA was used as a probe to screen a mouse genomic library (Mouse Genomic, 129 SVJ Library; Stratagene) in accordance with a manual attached thereto. A phage clone containing a sequence of approximately 17.9 kbp (corresponding to 90644 to 108544 in mouse-pub-genome sequence AC073775.2 containing the AGF gene) was isolated and subcloned into plasmid pBluescript (Stratagene). The obtained plasmid clone (pBN2) was digested with restriction enzymes SalI and MfeI to obtain the 5' long arm of approximately 6.2 kbp (containing 90664 to 96900 in mouse-pub-genome sequence ACO73775.2). Further, a PCR was carried out using the plasmid pBN2 as a template, together with a primer set [SEQ ID NO: 6 (CTAGACTAGTTGCAAAGGCGTGCGGCGG; artificial sequence) and SEQ ID NO: 7 (CTAGACTAGTGGATCCGCAGGCTTGCTTTGACTTAC; artificial sequence)] to obtain the 3' short arm of approximately 2.0 kbp (containing 105903 to 107914 in mouse-pub-genome sequence AC073775.2). The 5' long arm and the 3' short arm were inserted into the XhoI site and the XbaI site of plasmid pPNT [Cell, 1991, 65(7), 1153-1163], respectively, to construct the targeting vector.

### (2) Preparation of homologous recombinant ES cells

The obtained targeting vector was digested with restriction enzyme NotI, and introduced into ES cell line R1 (Proceedings of the National Academy of Sciences, Vol 90, 8424-8428, 1993) by electroporation. The ES cells were cultured in a medium containing G418 to obtain resistant strains. DNAs were extracted from ES cells, and clones in which only a desired homologous recombination occurred were identified by Southern blotting. More particularly, each DNA was digested with restriction enzyme HindIII, and analyzed by Southern blotting using, as a probe, a DNA consisting of the nucleotide sequence of SEQ ID NO: 8 
(GCCCATGGAGGGATTGTGCAGAGGCTCACGGGGCAGGTCACTGGCAGAGTGGAGTGTATGA
CCTGCGGCTGGGCCGTCGTGTAGTAGCCGTGTGGTGTGAACAGCAGCAGGAAGTGGAGGCTG
GACTGTCATCCAGAGACGGCAGGACGGCTCTGTCAACTTCTTCACCAACTGGCAGCACTACA
AGGTGTGTGCTTGTGGTGGGGGTGTCAGAGACTGCTGGCAGAGAGGACGCCCCCACCCTCT
TCCTCCTACCCTTCCAGGCGGGCTTTGGGCGTCCAGAAGGAGAATACTGGCTGGCCTGGAA
CCTGTGCATCAGGTGACAAGCCGTGGGACCACGAGCTGCTGATACTCCTAGAGGACTGGGG
GGGCCGTGCAGCACGCGCCCACTACGACAGCTTCTCCTTGGAGCCTGAGAGTGACCACTACC
GTCTGCGGCTTGGCCAGTACCACGGCGATGCCGGAGACTCCCTCTCTTGGCACAATGACAAA
ACCTTTCAGCACTGTGGATAGGGACAGAGACTCATATTCTG; mouse)
 containing exon 4 and exon 5 in AGF. As a result, a DNA fragment of 6.5 Kb was detected in homologous recombinant clones, in comparison with that of 4.6 Kb in the wild-type.

### (3) Preparation of AGF KO mice

The obtained ES cell line was microinjected into blastocysts prepared from BDF2 mice which were F2 hybrid mice of C57BL/6 and DBA/2 mice, and the manipulated eggs were transferred to a uterus, to obtain chimeric mice from the pregnant mice. The chimeric mice were mated with C57BL/6 mice to obtain heterozygous mice (hereinafter referred to as AGF heterozygous KO mice) having a mutated allele lacking in the initiation codon of AGF. The AGF heterozygous KO mice were mated with each other to obtain homozygous mice (hereinafter referred to as AGF homozygous KO mice). A PCR using genomic DNA isolated from the tail of each offspring mouse as a template was carried out to confirm the genotype thereof from the size of each DNA fragment obtained by the PCR, as described below. That is, the tail was treated with proteinase K, and a phenol/chloroform extraction was carried out to obtain DNA. The extracted DNA was collected by an isopropanol precipitation followed by an ethanol precipitation, and dissolved in a Tris-EDTA buffer (hereinafter referred to as TE solution). The following primers were designed on the basis of a sequence of the neomycin resistant gene and a genomic sequence to be deleted by targeting:

(neomycin resistant gene)
Forward primer: SEQ ID NO: 9 (5′-agaggctattcggctatgac-3′;
artificial sequence)

Reverse primer: SEQ ID NO: 10 (5′-caccatgatattcggcaagc-3′;
artificial sequence)

(genomic DNA)
Forward primer: SEQ ID NO: 11 (5'-tggcctctgttatcatgctc-3';
mouse)

Reverse primer: SEQ ID NO: 12 (5′-ctacctacatccactcctac-3′;
mouse)

The genomic DNA obtained from each offspring mouse was used together with the above primers and a DNA polymerase (ExTaq; Takara) to perform PCRs. In the PCRs, a thermal denature at 95°C for 5 minutes was carried out, a cycle composed of reactions at 95°C for a minute, at 60°C for a minute, and at 72°C for a minute was repeated 30 times, and an elongation reaction at 72°C for 7 minutes was carried out. The sizes of fragments amplified by the PCRs were analyzed. When an offspring mouse has the mutated allele, a band of 545 bp is detected in the PCR for detecting the neomycin resistant gene. When an offspring mouse has the wild-type allele, a band of 322 bp is detected in the PCR for detecting the genomic DNA containing mouse AGF exon 1. The genotype of each mouse was determined from the results. As a result, in the AGF homozygous KO mice, the band of the mutated allele was detected, but the band of the wild-type allele was not detected. In the AGF heterozygous KO mice, the band of the mutated allele and that of the wild-type allele were detected. In the wild-type mice (hereinafter referred to as littermate WT mice), the band of the mutated allele was not detected, but the band of the wild-type allele was detected.

Further, the genotype of each mouse was analyzed by Southern blotting described in Example 1(2). As a result, in the AGF homozygous KO mice, a band of 6.5 kbp derived from the mutated allele was detected. In the AGF heterozygous KO mice, a band of 6.5 kbp derived from the mutated allele and that of 4.6 kbp derived from the wild-type allele were detected. In the littermate WT mice, the band of 4.6 kbp derived from the wild-type allele was detected.

Furthermore, each blood sample collected from the AGF KO mice was allowed to stand at 37°C for 30 minutes, and centrifuged to obtain a serum as the supernatant. The serum was diluted to 1/20 with a lysis buffer [0.5 mol/L HEPES (pH7.2), 1% Triton X-100, 10% glycerol, 10 mmol/L Na₄P₂O₇, 0.1 mol/L NaF, 0.1 mmol/L Na₃VO₄, 4 mmol/L EDTA (pH 8), 0.05 mg/mL aprotinin, 1 mmol/L PMSF, 0.1 mmol/L leupeptin, and 0.025 mmol/L Pepstatin A], and further diluted with an equal volume of a 2×SDS sample buffer. Each sample (20 µL per lane) was subjected to 10% acrylamide gel electrophoresis, followed by Western blotting. In the Western blotting, TBS-T [20 mmol/L Tris-HCl (pH 7.5), 150 mmol/L NaCl, and 0.05%(w/v) Tween 20] containing 5% bovine serum albumin (BSA) was used as a blocking agent, an anti mouse AGF antibody (WO03/083114) was used as the first antibody, and an anti rabbit antibody (ALI3404; BIO SourCE) diluted to 1/5000 with TBS-T containing 3% BSA was used as the second antibody. The AGF band was not detected in the AGF homozygous KO mice, to confirm that AGF was deficient.

### Example 2: Preparation of CAG-AGF Tg mice

In this example, AGF transgenic mice (hereinafter referred to as CAG-AGF Tg mice) in which mouse AGF was systemically overexpressed under the control of a CAG (modified chicken beta-actin promoter with CMV-IE enhancer) promoter [GENE, 108(1991) 193-200] were prepared. A plasmid in which the CAG promoter (1.7 kb), a lox71 sequence, a blasticidin gene (bsr), a poly A signal sequence (0.5 kb), a lox P sequence, a mouse AGF cDNA sequence, and an IRES (internal ribosomal entry site)-β-geo-poly A sequence (4.5 kb) were inserted at the multicloning site of plasmid pBluescriptII KS(+) (Stratagene) in this order was prepared in accordance with the following procedures.

The full-length of mouse AGF cDNA prepared by the procedures described in WO03/083114 was used as a template, together with a primer set [SEQ ID NO: 13 (AGAAGCTTCACCATGGGGACCGCCAGGCTAC; artificial sequence) and SEQ ID NO: 14 (CCGTCGACATTAGATCTTCACAAGCGCACAAGCCGGGTC; artificial sequence)] to carry out a PCR. In the PCR, a reaction at 95°C for 10 minutes was carried out, and a cycle composed of reactions at 94°C for 15 seconds, at 60°C for 30 seconds, and at 72°C for 2 minutes was repeated 45 times. The obtained PCR product was subcloned into a pZErO-2 cloning vector (Invitrogen). The obtained plasmid was digested with restriction enzymes HindIII and SalI, and inserted between the HindIII and SalI sites of plasmid pBluescriptII SK (Stratagene) to construct plasmid pBS-mAGF containing the full-length of mouse AGF gene. To introduce the IRES-β-geo-poly A gene into the plasmid pBS-mAGF, plasmid pU-San (Hum Mol Genet 8:387-396 1999) carrying the IRES-β-geo-poly A gene was digested with restriction enzymes SalI and BglII, and the obtained IRES-β-geo-poly A gene was inserted between the BglII and SalI sites of pBS-mAGF to construct plasmid pBS-mAGF-βgeo containing the mouse AGF cDNA sequence and the IRES-β-geo-poly A sequence.

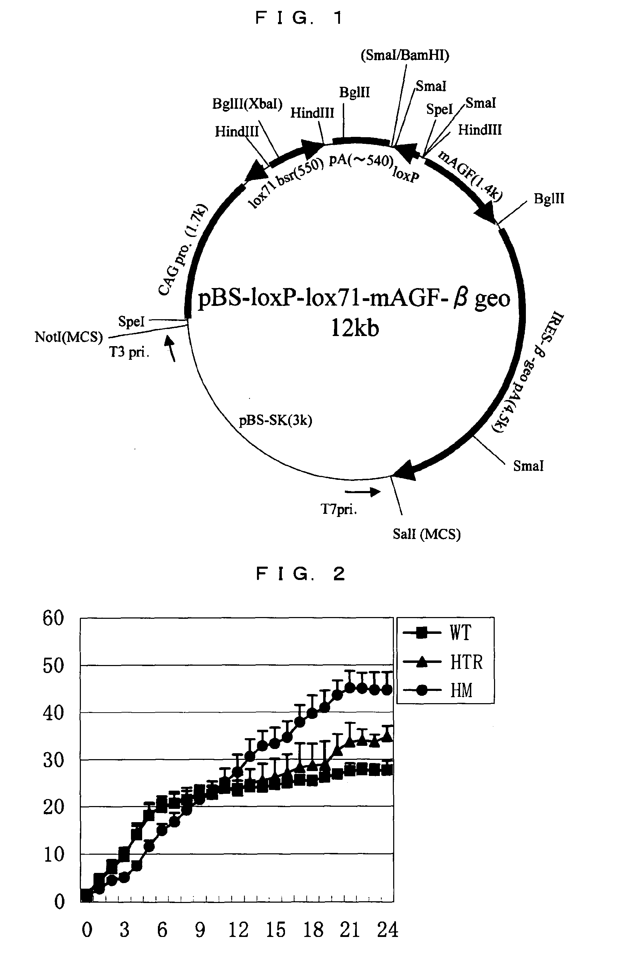

A plasmid in which bsr and the poly A signal sequence are interposed between the lox71 sequence and the loxP sequence by inserting the loxP sequence into the 3' side of the poly A signal of plasmid pCAGlox71bsr [Nucleic Acids Res. 1997; 25(4): 868-872] carrying the CAG promoter, the lox71 sequence, bsr, and the poly A signal sequence, was constructed as described below. That is, a phosphorylated fragment of 81 bp (SEQ ID NO: 15; GATCCGGAACCCTTAATATAACTTCGTATAATGTATGCTATACGAAGTTATTAGGTCCCTCG ACCTGCAGCCCGGGGGATC; artificial sequence) carrying loxP was inserted into the plasmid pCAGlox71bsr, which had been previously digested with restriction enzyme SmaI and had been treated with BAP (bacterial alkaline phosphatase), to construct plasmid loxP-lox71. To confirm the loxP sequence inserted in the plasmid loxP-lox71, a nucleotide sequence containing the inserted loxP sequence was sequenced using a T3 primer (SEQ ID NO: 16; AATTAACCCTCACTAAAGGG). As a result, the nucleotide sequence determined by the T3 primer accorded with that of SEQ ID NO: 15, and thus, it was confirmed that the loxP and lox71 sequences have the same direction. The plasmid loxP-lox71 was digested with restriction enzyme SpeI, and the obtained fragment containing the CAG promoter, the lox71 sequence, bsr, the poly A signal sequence, and the loxP sequence was inserted at the SpeI site of plasmid pBS-mAGF-βgeo to construct the desired plasmid pBS-loxP-lox71-mAGF-βgeo. The structure of the plasmid pBS-loxP-lox71-mAGF-βgeo is shown in Figure 1. The plasmid pBS-loxP-lox71-mAGF-βgeo was digested with restriction enzyme NotI to obtain a linearized DNA fragment containing the CAG promoter, the lox71 sequence, bsr, the poly A signal sequence, the loxP sequence, the mouse AGF cDNA sequence, and the IRES-β-geo-poly A sequence (pA).

The linearized DNA fragment was introduced into TT2 ES cells [Anal. Biochem., 1993, 214(1): 70-76] by electroporation (0.8 V, 3 µF). The ES cells were cultured in the presence of 4 µg/mL blasticidin, and cells in which the genes were introduced were selected to establish 20 clones. A CAG-Cre vector (Blood, 1326-1333, Vol.100, 2002) as a circular plasmid was introduced into each clone by electroporation (0.8 V, 3 µF).

When Cre recombinase expressed by the CAG promoter excises the gene between lox71 and loxP, AGF and β-geo will be expressed by the CAG promoter activity. To select clones expressing β-geo, the ES cells were cultured in the presence of G418 (200 µg/mL) to select cells having the genetic structure in which the region between lox71 and loxP were deleted. ES cells (15 clones) selected at this stage have the CAG promoter (1.7 kb), the lox71 sequence, the mouse AGF cDNA sequence, and the IRES-β-geo-poly A sequence (4.5 kb), and express AGF constitutively under the control of the CAG promoter. Each original clone before introducing the CAG-Cre vector (i.e., ES cells in which the region between lox71 and loxP was not deleted) was used as a negative control to confirm that AGF was expressed in the selected 15 clone by Western blotting using the anti AGF antibody described in Example 1(3). That is, ES cells were lysed with the lysis buffer, followed by an equal volume of the 2×SDS sample buffer, the obtained samples were subjected to Western blotting, and the expression of AGF was confirmed. From the ES cells expressing AGF, three lines (8-1, 8-2, and 9-1) were selected. The selected cell lines were microinjected into blastocysts, and the manipulated eggs were transferred to a uterus to obtain chimeric mice from the pregnant mice. The chimeric mice were mated with C57BL/6 mice to obtain transgenic mice expressing AGF constitutively under the control of the CAG promoter. To identify the transgenic mice, a PCR using genomic DNA isolated from the tail of each offspring mouse as a template was carried out, as described below. That is, the tail was treated with proteinase K, and a phenol/chloroform extraction was carried out to obtain DNA. The extracted DNA was collected by an isopropanol precipitation followed by an ethanol precipitation, and dissolved in a TE solution.

The following primers were designed on the basis of the mouse AGF cDNA sequence and a LacZ sequence:

(AGF)
Forward primer: SEQ ID NO: 17 (5'-cccactacgacagcttctcc-3';
mouse)

Reverse primer: SEQ ID NO: 18 (5′-agccgggtcaacataacagc-3′;
mouse)
(LacZ)

Forward primer: SEQ ID NO: 19 (5'-gcgttacccaacttaatcg-3';
artificial sequence)

Reverse primer: SEQ ID NO: 20 (5'-tgtgagcgagtaacaacc-3';
artificial sequence)

In PCRs using these primers, fragments of 325 bp and 320 bp are amplified from the introduced gene in PCRs for detecting the AGF cDNA and for detecting LacZ, respectively, and such fragments are not amplified from the mouse genomic DNA. The above primers and each genomic DNA prepared from the offspring mice were used to perform PCRs using a DNA polymerase (ExTaq; Takara). In the PCRs, a thermal denature at 94°C for 5 minutes was carried out, a cycle composed of reactions at 94°C for a minute, at 62°C for a minute and 30 seconds, and at 72°C for a minute and 30 seconds was repeated 28 times, and an elongation reaction at 72°C for 7 minutes was carried out. The sizes of fragments amplified by the PCRs were analyzed. As a result, the expected bands were detected in both PCRs for detecting the AGF cDNA and LacZ, with respect to the three lines. The result shows that a germ line transmission occurred in the three lines, and that the three lines are transgenic mice expressing AGF constitutively under the control of the CAG promoter.

### Example 3: Expression of AGF gene in CAG-AGF Tg mouse

In this example, the degree of the AGF gene expressed in the CAG-AGF Tg mouse prepared in Example 2 was analyzed. Total RNAs were prepared from the CAG-AGF Tg mouse and the littermate WT mouse [white adipose tissue (WAT), brown adipose tissue (BAT), cerebrum, cerebellum, hypothalamus, heart, liver, kidney, spleen, skeletal muscle, and pancreas] using a trizol reagent (Invitrogen). An commercially available RNA purification reagent (RNeasy; Qiagen) and DNase (Qiagen) were used to perform a DNase treatment and cleanup of the total RNAs. After the DNase treatment, 0.5 µg of the total RNAs were converted to cDNAs using superscript first-strand system for RT-PCR (LIFE TECHNOLOGIES).

Amounts of AGF and 18S ribosomal RNA (18SrRNA) expressed were determined by a quantitative PCR method. The 18SrRNA was used as an internal standard. The quantitative PCR was carried out by measuring an amount of real-time fluorescence using a sequence detection system (ABI PRISM 7900HT Sequence Detection System; Applied Biosystems). The above cDNAs were used as a template, and the following primers and a Taq Man probe designed for each gene were used as primers. Primers [SEQ ID NO: 21 (TCGTGTAGTAGCCGTGTGGTGT; mouse) and SEQ ID NO: 22 (CACCTGATGCACAGGTTCCA; mouse)] and a commercially available PCR reagent (SYBR Green PCR Master Mix; Applied Biosystems) were used to carry out a PCR for measuring an amount of the AGF gene expressed. Primers [SEQ ID NO: 23 (TGGTTGATCCTGCCAGTAG; mouse) and SEQ ID NO: 24 (CGACCAAAGGAACCATAACT; mouse)], a Taq Man probe [SEQ ID NO: 25 (CCGGTACAGTGAAACTGCGAATG; mouse)], and a commercially available PCR reagent (TaqMan Universal PCR Master Mix; Applied Biosystems) were used to carry out a PCR for measuring an amount of the 18SrRNA expressed.

In the PCR, an initial denaturing reaction at 95°C for 10 minutes was carried out, and a cycle composed of reactions at 94°C for 15 seconds and at 60°C for 60 seconds was repeated 45 times. Standard curves for calculating amounts of genes expressed were prepared by using the above cDNAs or mouse genomic DNA as a template. Amounts of genes expressed were calculated as relative values between samples.
As a result, it was revealed that an amount of the AGF gene expressed in tissues of the CAG-AGF Tg mouse was increased in comparison with that in tissues of the WT mouse. In particular, increased expressions were remarkably observed in skeletal muscle, BAT, and heart.

### Example 4: Changes in body weight of genetically modified mice

### (1) Changes in body weight of CAG-AGF Tg mice

Two generations of backcrosses of the CAG-AGF Tg mouse with C57BL/6 were carried out to obtain CAG-AGF Tg mice (F2). The CAG-AGF Tg mice and the littermate WT mice were normally bred with a normal diet (CE-2; CLEA Japan). When the body weights of 6-week-old female mice were measured and compared, those of the CAG-AGF Tg mice and the WT mice were 15.7 g ± 0.8 g (SD) and 17.8 g ± 0.5 g (SD), respectively. When those of 12-week-old female mice were measured and compared, those of the CAG-AGF Tg mice and the WT mice were 19.5 g ± 0.7 g (SD) and 23.5 g ± 2.5 g (SD), respectively. It was found from the results that the body weight of the CAG-AGF Tg mouse was lighter than that of the WT mouse.

Changes in body weight when the above mice were bred with a high fat diet were examined. The mice were normally bred with a high fat diet (HFD-32; CLEA Japan) for 12 weeks, and changes in body weight were analyzed. As a result, the amounts of body weights increase for 12 weeks were 7.1 g ± 1 g (SD) in CAG-AGF Tg mice and 21.8 g ± 4 g (SD) in WT mice, respectively. The results show that when the CAG-AGF Tg mice were bred with a normal diet, an increase in body weight was suppressed, and that when the CAG-AGF Tg mice were bred with a high fat diet, an increase in body weight was remarkably suppressed. It was found from the results that AGF exhibits an activity of suppressing an increase in body weight.

### (2) Changes in body weight of AGF KO mice

The AGF homozygous KO mice, the AGF heterozygous KO mice, and the littermate WT mice were normally bred with a normal diet. Each body weight was measured every week until 24-week-old. The results are shown in Figure 2. As shown in figure 2, the body weight of the AGF homozygous KO mice was higher than that of the WT mice from approximately 12-week-old, and an increase in body weight of the AGF homozygous KO mice continued to become remarkable obese. The AGF heterozygous mice exhibited an intermediate phenotype between those of the AGF homozygous KO mice and the littermate WT mice.

### Example 5: Changes in organ weight of genetically modified mice

### (1) Changes in organ weight in CAG-AGF Tg mice

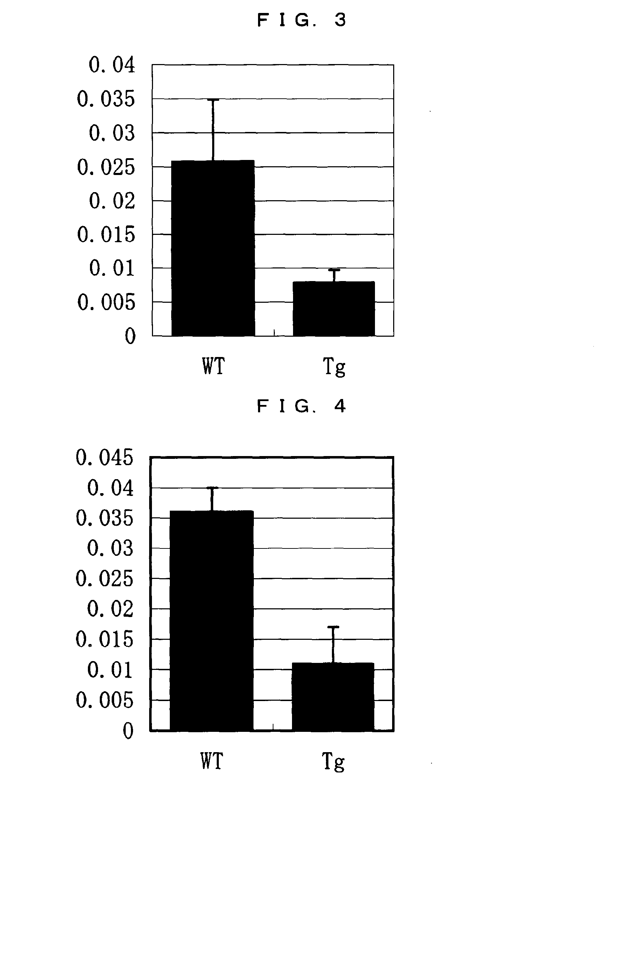

As described above, it was found that an increase in body weight was suppressed in the CAG-AGF Tg mouse. In this example, the weights of various organs were measured to reveal the mechanism. Each organ [genital fat pads (WAT), brown adipose tissue (BAT), liver, heart, kidney, and spleen] was obtained from the CAG-AGF Tg mice and the littermate WT mice, and the weight of each organ per body weight was measured. The measurement was carried out using 12-week-old mice bred with a normal diet and mice bred with a high fat diet for 12 weeks (from 12 week-old to 24-week-old). As a result, no changes were observed in BAT, liver, heart, kidney, and spleen between the mice (i.e., CAG-AGF Tg mice and WT mice) bred with a normal diet or a high fat diet. In contrast, the weight per body weight of genital fat pads (white adipose tissue) in the CAG-AGF Tg mice bred with a normal diet or a high fat diet was decreased in comparison with that in the WT mice. The results show that, in the CAG-AGF Tg mouse, an increase in the weight of WAT was suppressed, and thus, an increase in the body weight thereof was suppressed. That is, it was found that AGF does not act on the weights of organs other than WAT, but suppresses an increase in the weight of adipose tissue accompanied by obesity. The results in WAT are shown in Figure 3 (normal diet) and Figure 4 (high fat diet), respectively.

### (2) Changes in organ weight in AGF KO mice

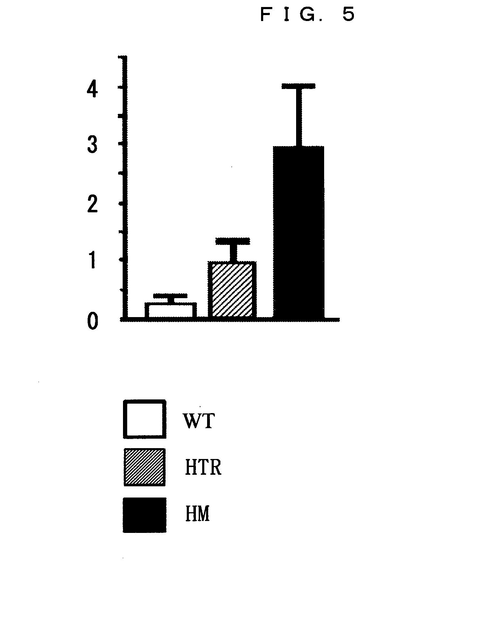

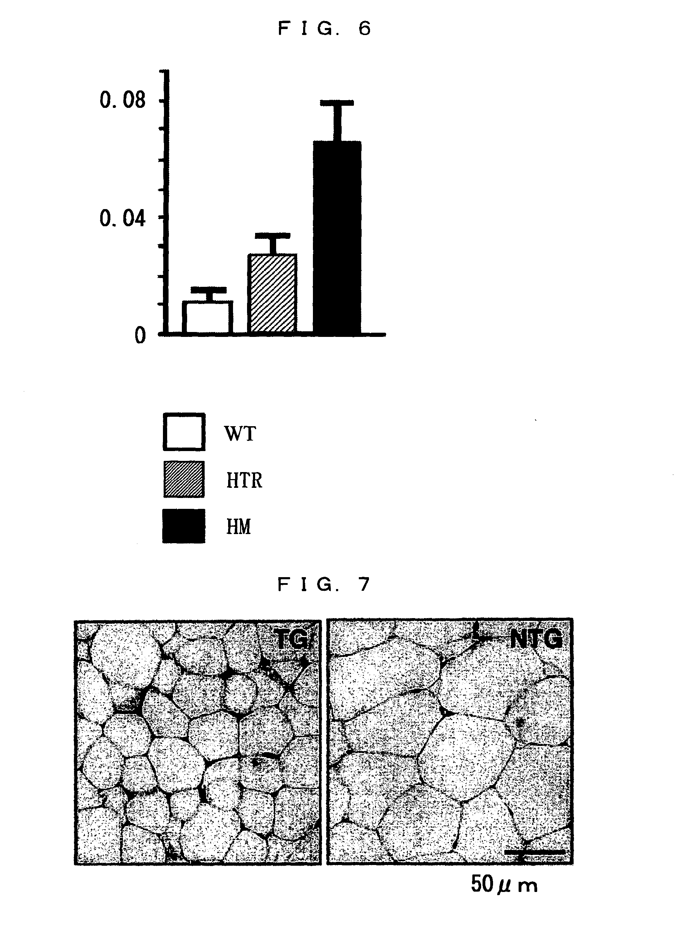

As described above, it was found that the body weight was increased in the AGF KO mouse. In this example, the weights of various organs were measured to reveal the mechanism. Each organ [genital fat pads (WAT), brown adipose tissue (BAT), liver, heart, kidney, and spleen] was obtained from 20-week-old female AGF homozygous KO mice, AGF heterozygous mice, and littermate WT mice bred with a normal diet, and the weight of each organ per body weight was measured. As a result, no changes were observed in BAT, liver, heart, kidney, and spleen among the AGF homozygous KO mice, the AGF heterozygous mice, and the littermate WT mice. In contrast, the weight per body weight or per mouse of genital fat pads in the AGF homozygous mice was increased in comparison with that in the WT mice. The AGF heterozygous mice exhibited intermediate organ weights between those of the AGF homozygous KO mice and the WT mice. The results show that, in the AGF KO mouse, the weight of WAT such as genital fat pads was increased, and thus, the body weight thereof was increased. That is, it was found that the AGF KO mouse exhibits phenotypes opposite to the CAG-AGF Tg mouse, and that AGF does not act on the weights of organs other than WAT, but suppresses an increase in the weight of adipose tissue. The results in genital fat pads (WAT) are shown in Figure 5 (weight of WAT) and Figure 6 (weight of WAT/body weight), respectively.

### Example 6: Changes in form of adipocytes in genetically modified mice

### (1) Changes in form of adipocytes in CAG-AGF Tg mice

It is known that a high fat diet increases the weight of WAT and hypertrophy of adipocytes. It is known that the hypertrophy of adipocytes is involved in deteriorating diabetes [IGAKU NO AYUMI, 192, 513-518, 2000; and IGAKU NO AYUMI, 192, 541-545, 2000]. Therefore, the forms of adipocytes in the CAG-AGF Tg mice were analyzed in this example, as described below. Each fat tissue was obtained from CAG-AGF Tg mice and littermate WT mice bred with a high fat diet for 12 weeks from 12-week-old to 24-week-old. Each tissue was fixed with a 10% formalin neutral buffer solution (Wako) and embedded in paraffin. Sliced sections were prepared and a hematoxylin and eosin (H&E) stain was carried out. The result is shown in Figure 7. In the littermate WT mouse (NTG), adipocytes became hypertrophied. In the CAG-AGF Tg mouse (TG), the hypertrophy of adipocytes was suppressed and the sizes thereof were maintained as a normal size.

### (2) Changes in form of adipocytes in AGF KO mice

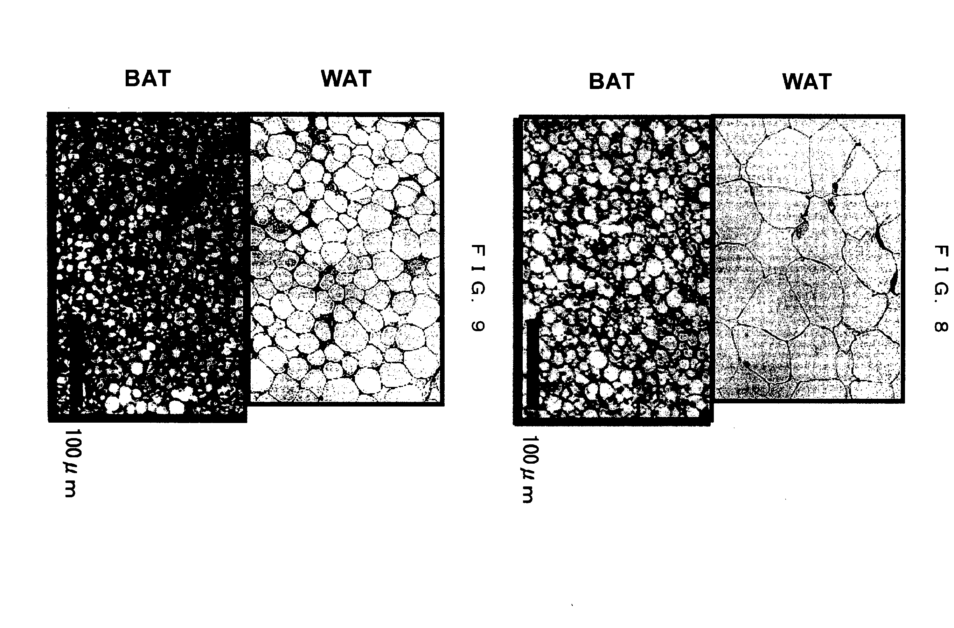

It is known that adipocytes become hypertrophied in model mice for diabetes or obesity, accompanied by an increase in the weight of adipocytes [Diabetologia, 14(3), 141-148, 1978]. It is known that the hypertrophy of adipocytes is involved in deteriorating diabetes, and thus, the forms of adipocytes in the AGF KO mice were analyzed in this example, as described below. Each genital fat pads (WAT) and brown fat tissue (BAT) were obtained from the AGF homozygous mice and the littermate WT mice. Each tissue was fixed with a 10% formalin neutral buffer solution and embedded in paraffin. Sliced sections were prepared and a hematoxylin and eosin (H&E) stain was carried out. The results are shown in Figure 8 (AGF homozygous KO mouse) and Figure 9 (littermate WT mouse), respectively. It was found that adipocytes in WAT of the littermate WT mice had a normal size, and that adipocytes in WAT of the AGF homozygous KO mice became hypertrophied. Further, an accumulation of fat in BAT of the AGF homozygous KO mice was observed in comparison with that of the littermate WT mice.

### Example 7: Changes in triglyceride content in tissues of genetically modified mice

### (1) Changes in triglyceride content in tissues of CAG-AGF Tg mice

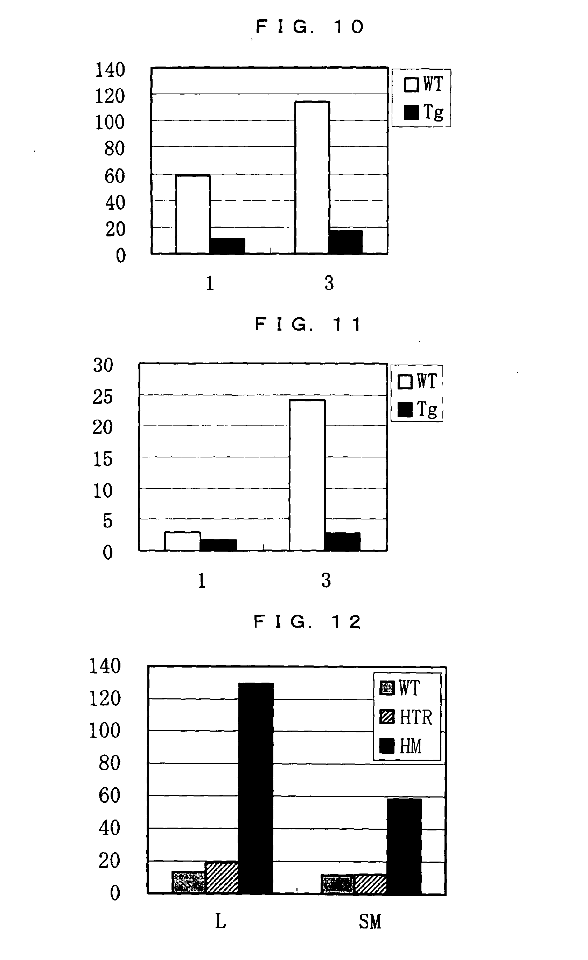

It is known that obesity causes not only an increase in fat tissues, but also an increase in triglyceride (TG) content in skeletal muscles or liver (Nippon Rinsho, 1995, vol. 53, Special Issue in 1995, Himansho, p. 354-p358). In this example, TG contents in skeletal muscles (gastrocnemial muscles) and liver of the CAG-AGF Tg mice were analyzed, as described below. The CAG-AGF Tg mice (Tg) and the littermate WT mice (WT) were bred with a high fat diet (HFD-32; CLEA Japan) for a month or three months. A chloroform-methanol solution was used to extract TG from skeletal muscles and liver of each mouse (seikagakujikkenkouza 3, shishitsunokagaku, tokyo kagaku doujin). A concentration of each extracted TG was measured using a kit (Triglyceride E test Wako; Wako) to determine TG contents in the tissues. The results are shown in Figure 10 (liver) and Figure 11 (skeletal muscles), respectively. It was found that each TG content in tissues (skeletal muscles or liver) of the CAG-AGF Tg mice was decreased in a comparison with that in the WT mice. It was found that AGF exhibits an activity of decreasing TG contents in skeletal muscles or liver. In this connection, it is known that the TG contents in skeletal muscles or liver are increased by obesity.

### (2) TG contents in tissues of AGF KO mice

TG contents in skeletal muscles (gastrocnemial muscles) and liver of the AGF KO mice were analyzed. The method described in Example 7(1) was repeated, except for that the AGF KO mice and the littermate WT mice were used, to extract TG therefrom. A concentration of each extracted TG was measured using a kit (Triglyceride E test Wako; Wako) to determine TG contents in the tissues. The results are shown in Figure 12. It was found that TG contents in skeletal muscles and liver of the AGF KO mice were remarkably increased in comparison of that in the WT mice. It was found that the AGF KO mouse exhibits phenotypes opposite to the CAG-AGF Tg mouse, and that AGF exhibits an activity of decreasing TG contents in skeletal muscles or liver.

### Example 8: Changes in blood glucose level and blood insulin concentration in AGF KO mice

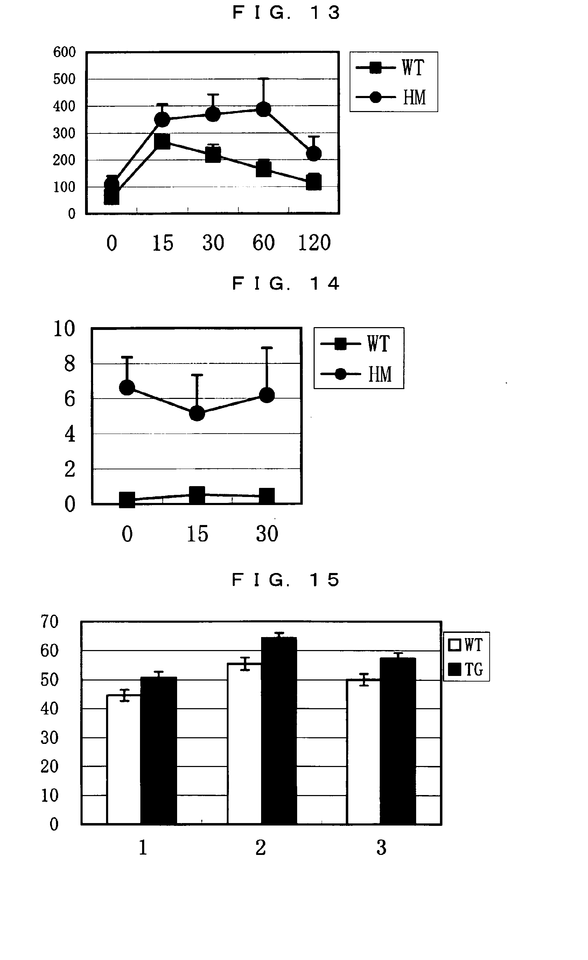

A glucose tolerance test for the AGF homozygous KO mice and the littermate WT mice was carried out to analyze a blood glucose level and a concentration of blood insulin, as described below. The mice were made to fast for 16 hours, and 1 g/kg of D-glucose was intraperitoneally administered. Blood was taken from ophthalmic veins before the administration, and at 15, 30, 60, and 120 minutes after the administration. The blood glucose level was measured using GLUTEST ACE (SANWA KAGAKU KENKYUSHO), and the concentration of blood insulin was measured using a RIA2 antibody method (SRL). The results are shown in Figure 13 (blood glucose value) and Figure 14 (insulin in sera), respectively.

In the WT mice, the blood glucose level increased by the glucose administration began to decrease at 30 minutes after the administration. In the AGF homozygous mice, the blood glucose level was greatly increased by the glucose administration, and the elevated blood glucose value did not begin to decrease at 60 minutes after the administration. It was found from the results that the AGF homozygous KO mice exhibited an abnormality in glucose tolerance. With respect to the concentration in blood insulin, it was increased by the glucose administration in the WT mice, whereas the concentration of blood insulin in the AGF homozygous KO mice was remarkably high before the glucose administration. This shows that the AGF homozygous KO mice suffered from hyperinsulinemia. From the results, it was found that the AGF homozygous KO mouse deficient in the AGF gene suffered from diabetes, and that AGF exhibits an antidiabetic activity.

### Example 9: Oxygen consumption in CAG-AGF Tg mice

The oxygen consumption in the CAG-AGF Tg mice and the littermate WT mice was measured in this example. An apparatus for measuring oxygen consumption (OXYMAX; Columbus Instruments) was used to measure oxygen consumption in fasting mice for 24 hours in accordance with a manual attached to the apparatus. Oxygen consumption in 14 CAG-AGF Tg mice and 14 littermate WT mice was measured to determine and prepare oxygen consumption for a 12-hour light period (7:00~19:00), that for a 12-hour dark period (19:00~7:00), and that for 24 hours. The results are shown in Figure 15. The oxygen consumption (VO₂) in the CAG-AGF Tg mice was higher than that in the WT mice in any time zone. It was found that AGF exhibits an activity for promoting oxygen consumption. Because oxygen consumption correlates with energy consumption, the promotion of oxygen consumption exhibits an antiobesity activity [FEBS Letters, 491(1-2): 154-158, 2001]. It was found that AGF exhibits an activity of promoting oxygen consumption. The result supports the antiobesity activity of AGF.

### Example 10: Changes in genes expressed in tissues of CAG-AGF Tg mice

In this example, changes in a UCP (Uncoupling Protein) gene and a PPAR (Peroxisome Proliferator-Activated Receptor) gene expressed in brown adipose tissue (BAT) and skeletal muscles of the CAG-AGF Tg mice were analyzed. In accordance with the procedures described in Example 3, total RNAs were prepared from BAT and skeletal muscles of the CAG-AGF Tg mice and the littermate WT mice, and were treated with DNase, and cDNAs were synthesized. Amounts of UCP1, UCP3, PPAR-α, PPAR-δ, and β-actin expressed were determined by the quantitative PCR method, described in Example 3. Primers shown in Table 1 were used in the quantitative PCR. Further, as commercially available PCR reagents, SYBR Green PCR Master Mix (Applied Biosystems) was used for β-actin, and TaqMan Universal PCR Master Mix (Applied Biosystems) was used for UCP1, UCP3, PPAR-α, and PPAR-δ.

**[Table 1]**

| Genes | Forward primer | Reverse primer | TaqMan primer |
|---|---|---|---|
| UCP 1 | SEQ ID NO: 26 | SEQ ID NO: 27 | SEQ ID NO: 28 |
| UCP 3 | SEQ ID NO: 29 | SEQ ID NO: 30 | SEQ ID NO: 31 |
| PPAR-α | SEQ ID NO: 32 | SEQ ID NO: 33 | SEQ ID NO: 34 |
| PPAR-δ | SEQ ID NO: 35 | SEQ ID NO: 36 | SEQ ID NO: 37 |
| β-actin | SEQ ID NO: 38 | SEQ ID NO: 39 | Not used |

As a result, it was found that the expression of UCP1 was induced in BAT of the CAG-AGF Tg mice, and that the expressions of UCP3, PPAR-α, and PPAR-δ were induced in skeletal muscles of the CAG-AGF Tg mice. That is, it was found that AGF induces the expression of UCP1 in BAT and those of UCP3, PPAR-α, and PPAR-δ in skeletal muscles. Accordingly, it was revealed that AGF promotion in expression of UCP promoting heat consumption and in expression of PPAR promoting heat consumption or lipid metabolism is one of the mechanisms of AGF activities for promoting oxygen consumption, suppressing an increase in body weight, and suppressing an increase in the weight of adipose tissues.

### Referential example 1: Expression and purification of mouse AGF and human AGF

The human AGF and the mouse AGF were expressed and purified in accordance with the procedures described in WO03/083114 (Example 19) as described below. That is, DNA fragments of approximately 1.4 kbp (human) and approximately 1.3 kbp (mouse) were independently inserted into plasmid pcDNA-Signal-FLAG. Each resulting expression plasmid was introduced into HEK293 cells. Each culture supernatant of the cells expressing the human AGF or the mouse AGF was purified by affinity chromatograph using anti FLAG-M2 monoclonal antibody agarose affinity gel (Sigma) to obtain human and mouse recombinant AGF proteins.

### Referential example 2: Changes in genes expressed in C2C12 cells differentiated into skeletal muscle by AGF stimulation

After C2C12 cells (obtained from ATCC) were cultured to the confluent conditions, the culture medium was changed from DMEM with 10% fetal calf serum (FCS) to DMEM with 2.5% horse serum, and the cultivation was further continued for 8 days to differentiate the cells to skeletal muscle cells. After starvation of C2C12 cells differentiated into skeletal muscle for 12 hours, 3 µg/mL of human or mouse AGF recombinant protein were added or not added to the cells, and the cells were allowed to stand for 4, 8, 12, and 24 hours thereafter. Total RNAs were prepared from AGF-stimulated cells and nonstimulated cells, and treated with DNase, and cDNAs were synthesized. Amounts of PPAR-α, PGC-1α (Peroxisome Proliferator-Activated Receptor-coactivator 1 alpha), and CYP (cyclophilin) genes expressed were measured by the quantitative PCR described in Example 10. Primers listed in Tables 1 and 2 were used in the quantitative PCR. As commercially available PCR reagents, SYBR Green PCR Master Mix (Applied Biosystems) was used for CYP, and TaqMan Universal PCR Master Mix (Applied Biosystems) was used for PPAR-α and PGC-1α. An amount of a LCAD (long-chain acyl-CoA dehydrogenase) gene expressed was measured by using a commercially available assay system (Assay on demand; Applied Biosystems) and TaqMan Universal PCR Master Mix (Applied Biosystems) (Assay ID: Mm00599660_m1). The procedures described in Example 10 were repeated, except that reagents contained in the above purchased assay system were used as primers and PCR reaction solutions, in accordance with attached protocols.

**[Table 2]**

| Genes | Forward primer | Reverse primer | TaqMan primer |
|---|---|---|---|
| PGC-1α | SEQ ID NO: 40 | SEQ ID NO: 41 | SEQ ID NO: 42 |
| CYP | SEQ ID NO: 43 | SEQ ID NO: 44 | Not used |

As a result, it was found that the stimulation of the human or mouse AGF promotes expressions of the PPAR-α, PGC-1α, and LCAD genes. It shows that AGF promotes heat consumption and fatty acid oxidation in skeletal muscle cells. It is known that PPAR-α or PGC-1α promotes lipid metabolism or heat consumption [Acta Physiologica Scandinavica. 178(4):425-434, 2003; and Endocrine Reviews 24(1):78-90, 2003]. AGF promoted the expressions of PPAR-α, LCAD, and PGC-1α, and thus, the result supports the antiobesity activity of AGF.

### Example 11: Cloning of upstream sequence of hAGF mRNA transcriptional region

### (1) Cloning of sequences of approximately 200, 300, 400, 600, and 800 bp upstream of the hAGF mRNA transcriptional region

Human genome (Genomic DNA; Clontech) was used as a template to carry out PCR with DNA polymerase (TaKaRa LA taq™; Takara). In the PCR, a reaction at 95°C for 2 minutes was carried out, a cycle composed of reactions at 94°C for 30 seconds, at 60°C for 30 seconds, and at 72°C for a minute was repeated 40 times, and a reaction at 72°C for 5 minutes was carried out. As primer sets, five primer sets (SEQ ID NOS: 45 and 46, SEQ ID NOS: 47 and 46, SEQ ID NOS: 48 and 46, SEQ ID NOS: 49 and 46, and SEQ ID NOS: 50 and 46) were used. Fragments of approximately 200, 300, 400, 600, and 800 bp obtained by using the above primer sets were independently subcloned into a cloning vector (pCR2.1-TOPO; Invitrogen). Each subclone was digested with restriction enzymes KpnI and NheI, and the obtained fragments (nucleotides 2790-3001, 2705-3001, 2604-3001, 2406-3001, and 2206-3001 of SEQ ID NO: 1) were independently inserted into vector pGV-B2 for a luciferase assay system (PicaGene Vector 2 basic vector; Toyo Ink) previously digested with KpnI and NheI, to obtain pGV-hAGFpro200(N6), pGV-hAGFpro300(N6), pGV-hAGFpro400(N6), pGV-hAGFpro600(N6), and pGV-hAGFpro800(N6).

As described above, nucleotide sequences consisting of nucleotides 2790-3001, 2705-3001, 2604-3001, 2406-3001, and 2206-3001 of SEQ ID NO: 1, i.e., regions of approximately 200, 300, 400, 600, and 800 bp upstream of a human AGF mRNA transcriptional region, were cloned, and plasmids capable of measuring promoter activities thereof were constructed.

### (2) Cloning of sequences of approximately 1, 1.3, and 3 kbp upstream of the hAGF mRNA transcriptional region

Cloning of sequences of approximately 1 kbp and 1.3 kbp upstream of the hAGF mRNA transcriptional region was carried out by PCR, as described below. A forward primer consisting of the nucleotide sequence of SEQ ID NO: 51 and a reverse primer consisting of the nucleotide sequence of SEQ ID NO: 52 were used for cloning the region of approximately 1 kb. A forward primer consisting of the nucleotide sequence of SEQ ID NO: 53 and a reverse primer consisting of the nucleotide sequence of SEQ ID NO: 52 were used for cloning the region of approximately 1.3 kb. Human genome (Genomic DNA; Clontech) was used as a template to carry out PCR with DNA polymerase (TaKaRa LA taq™; Takara) and the above primer sets. In the PCR, a reaction at 95°C for 2 minutes was carried out, a cycle composed of reactions at 94°C for 30 seconds, at 60°C for 30 seconds, and at 72°C for 3 minutes was repeated 45 times, and a reaction at 72°C for 5 minutes was carried out. Obtained fragments of approximately 1 kbp and 1.3 kbp were independently subcloned into a cloning vector (pCR-XL-TOPO; Invitrogen). Each subclone was digested with restriction enzymes KpnI and NheI, and the obtained fragments (nucleotides 2021-3028 and 1640-3028 of SEQ ID NO: 1) were independently inserted into vector pGV-B2 previously digested with KpnI and NheI, to obtain pGV-hAGFpro1k(N4) and pGV-hAGFpro1.3k(N4).

Further, human genome (Genomic DNA; Clontech) was used as a template to carry out PCR with DNA polymerase (TaKaRa LA taq™; Takara) and a primer set of a forward primer consisting of the nucleotide sequence of SEQ ID NO: 54 and a reverse primer consisting of the nucleotide sequence of SEQ ID NO: 55. In the PCR, a reaction at 95°C for 2 minutes was carried out, a cycle composed of reactions at 94°C for 30 seconds, at 63°C for 30 seconds, and at 72°C for 2 minutes and 30 seconds was repeated 45 times, and a reaction at 72°C for 5 minutes was carried out

The resulting fragment of approximately 2 kb was subcloned into a cloning vector (pCR-XL-TOPO; Invitrogen). The obtained subclone was digested with KpnI and XmaI to obtain a fragment containing the nucleotide sequence consisting of nucleotides 1-1768 of SEQ ID NO: 1. The pGV-hAGFpro1.3k(N4) plasmid contains two XmaI recognition sites, i.e., a XmaI recognition site in the sequence upstream of the hAGF mRNA transcriptional region and a XmaI recognition site in the multicloning site of the pGV-B2 vector. Therefore, 8.7 µg of pGV-hAGFpro1.3k(N4) was digested with 5 units of XmaI for 1 to 10 minutes to prepare a mixture of plasmids cleaved at 0, 1, or 2 sites. The plasmid cleaved at only one site was separated and extracted by electrophoresis. The obtained plasmid was digested with KpnI, and a fragment of 6.1 kbp (determined by electrophoresis) was taken to obtain a plasmid in which the XmaI site in the cloning site of pGV-B2 was not cleaved and the XmaI site in the hAGF promoter region was cleaved. To the plasmid, the fragment containing the nucleotide sequence consisting of nucleotides 1-1768 of SEQ ID NO: 1, obtained by the above-described procedure (i.e., treatment with KpnI and XmaI), was inserted to obtain pGV-hAGFpro3k(N4).

The plasmid pGV-hAGFpro800(N6) was digested with SnaBI and XbaI to obtain a fragment (N6 fragment) of approximately 2 kbp. The plasmids pGV-hAGFpro1k(N2), pGV-hAGFpro1.3k(N4), and pGV-hAGFpro3k(N4) were digested with SnaBI and XbaI to remove the excised fragment of approximately 2 kbp. The N6 fragment was inserted thereinto to obtain pGV-hAGFhAGFpro1k(N6), pGV-hAGFpro1.3k(N6), and pGV-hAGFpro3k(N6). These obtained plasmids contain a hAGF promoter region having nucleotide sequence consisting of nucleotides 2021-3001, 1640-3001, or 1-3001 of SEQ ID NO: 1 in the reporter plasmid.

As described above, nucleotide sequences consisting of nucleotides 2021-3001, 1640-3001, and 1-3001 of SEQ ID NO: 1, i.e., regions of approximately 1 kbp, 1.3 kbp, and 3 kbp upstream of the human AGF mRNA transcriptional region, were cloned, and plasmids capable of measuring promoter activities thereof were constructed.

### Example 12: Analysis of DNA sequence of human AGF promoter region

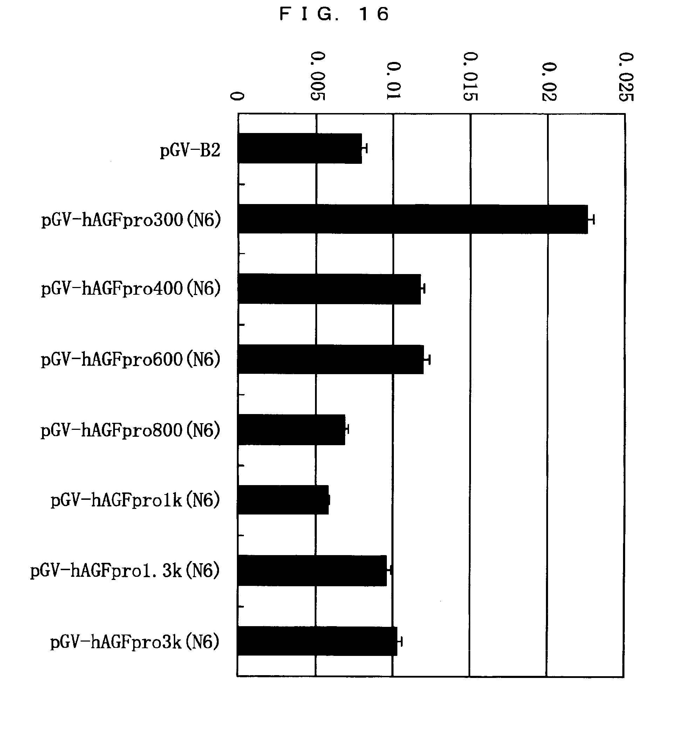

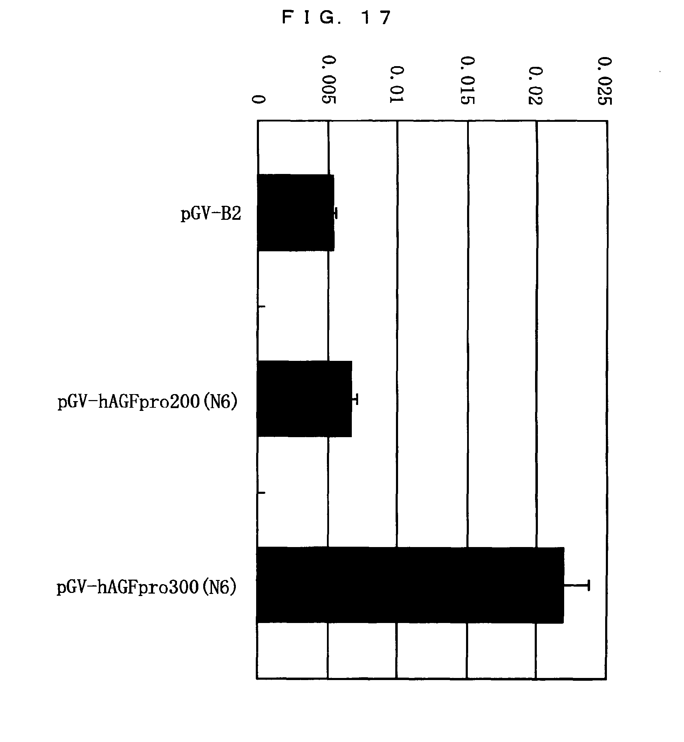

293EBNA cells (Invitrogen) cultured in Dulbecco's modified Eagle's medium (DMEM) supplemented with 10% fetal calf serum, 100 µg/mL penicillin, and 100 µg/mL streptomycin were transfected with i) pGV-B2 (control vector), or plasmids obtained in Example 11 [i.e., pGV-hAGFpro200(N6), pGV-hAGFpro300(N6), pGV-hAGFpro400(N6), pGV-hAGFpro600(N6), pGV-hAGFpro800(N6), pGV-hAGFprolk(N6), pGV-hAGFpro1.3k(N6), or pGV-hAGFpro3k(N6)] and ii) a β-gal expressing plasmid (pCH110; Amersham pharmacia biotech), using a transfection reagent (FuGene-6; Nippon Roche). A luciferase activity after 48-hour cultivation under conventional culture conditions was measured by a commercially available measuring kit (PicaGene luminescence kit; Toyo Ink). The measuring values were compensated on the basis of activities of β-gal expressed by the co-transfected β-gal expressing plasmid. The β-gal activity was measured by a commercially available measuring kit (Galacto-Light Plus kit; Roche). As a result, no increased luciferase activities were observed in cells transfected with plasmids containing DNAs of approximately 400 bp to 3 kbp [pGV-hAGFpro400(N6), pGV-hAGFpro600(N6), pGV-hAGFpro800(N6), pGV-hAGFpro1k(N6), pGV-hAGFpro1.3k(N6), or pGV-hAGFpro3k(N6)]. Unexpectedly, a remarkably increased luciferase activity, which was not observed in cells transfected with the control vector, was observed in cells transfected with the plasmid pGV-hAGFpro300(N6) containing the shorter DNA of approximately 300 bp (Figure 16). No increased luciferase activity was observed in cells transfected with the plasmid pGV-hAGFpro200(N6) containing the further shorter DNA of approximately 200 bp (Figure 17). The results show that a promoter activity is located in the region of approximately 300 bp consisting of the nucleotide sequence 2705-3001 of SEQ ID NO: 1, and that a silencer sequence capable of suppressing the promoter activity is located in the sequence upstream of the above promoter region of approximately 300 bp.

The present inventors constructed a reporter assay utilizing the DNA sequence of approximately 300 bp consisting of the nucleotide sequence consisting of nucleotides 2705-3001 of SEQ ID NO: 1, and found that the region contains the AGF promoter activity. An antiobesity agent, an antidiabetic agent, and/or a hypolipidemic agent, which promote the promoter activity of the AGF gene and promote the expression of AGF, may be screened by the assay system, preferably by bringing a test compound into contact with cells transfected with pGV-hAGFpro300 (N6) and analyzing the change in the luciferase activity.

### Referential example 3: Cloning of mouse AGF

The mouse AGF was amplified by using a forward primer consisting of the nucleotide sequence of SEQ ID NO: 56 and a reverse primer consisting of the nucleotide sequence of SEQ ID NO: 57. In the PCR, Pyrobest DNA polymerase (Takara) was used, and a cycle consisting of reactions at 98°C for 20 seconds, at 64°C for 30 seconds, and at 74°C for 3 minutes was repeated 35 times in the presence of 5% formamide, to amplify a DNA fragment of approximately 1.5 kbp. The fragment was cloned into a pCR2.1 plasmid (Invitrogen) to obtain plasmid pCR2.1-mNew. The nucleotide sequences of the obtained clones were analyzed by a DNA sequencer (ABI377 DNA Sequencer; Applied Biosystems) utilizing a dideoxytermination method. The determined sequence is shown as SEQ ID NO: 58. The sequence contains an open reading frame consisting of 1374 nucleotides (nucleotides 1-1374 of SEQ ID NO: 58). The amino acid sequence (457 amino acids) deduced from the open reading frame is shown as SEQ ID NO: 59. The mouse AGF contains a signal sequence (-24 to -1) at the N-terminus. The signal sequence is cleaved when AGF is secreted to the outside of cells. Mouse matured AGF consisting of the amino acid sequence consisting of amino acids 1-433 of SEQ ID NO: 59 after cleaving the signal sequence exhibits physiological activities.

The sequence (mouse) consisting of the amino acid sequence consisting of amino acids 1-433 of SEQ ID NO: 59 has a 76% homology with that (amino acids 21-470 in Genbank accession No. NP_114123) of human AGF known as human NL8/NEW, angiopoietin-like 6, or angiopoietin-related protein 5. In particular, a fibrinogen domain at the C-terminal side has a high homology (89%). In this connection, it is known that the fibrinogen domain plays an important role in the activities of proteins belonging to an angiopoietin family [William N. Procopio et al., J.Biol.Chem. 274: 30196-30201 (1999)]. From such a high homology in the region which is considered to be important in maintaining the activities, it is considered that the mouse AGF of SEQ ID NO: 58 is a counterpart of the human AGF and that the mouse AGF and the human AGF have the same activities. In this connection, the homologies between amino acid sequences were calculated by a BLAST search algorithm. More particularly, it may be calculated using a bl2seq program (Tatiana A. Tatusova and Thomas L. Madden, FEMS Microbiol. Lett., 174, 247-250, 1999) in a BLAST package (sgi32bit edition, version 2.0.12; obtained from NCBI) in accordance with a default parameter. As a pairwise alignment parameter, a program "blastp" is used. Further, "0" as a Gap insertion cost value, "0" as a Gap elongation cost value, "SEG" as a filter for a Query sequence, and "BLOSUM62" as a Matrix are used, respectively.

### Referential example 4: Preparation of OP9 cell line expressing AGF

Plasmid pCR2.1-mNew prepared in Referential example 3 was digested with restriction enzymes XbaI and SpeI to obtain a fragment of 1.4 kbp containing the mouse AGF gene. The fragment was inserted into pEF-BOS-neo [Mizushima, S., & Nagata, S. Nucleic Acids Res. 18: 5322 (1990)] , which had been previously digested with XbaI and treated with BAP, to prepare an expression vector pEF-BOS-mAGF for expressing mouse AGF. Fugene6 (Roche Diagnostics) was used in accordance with a protocol attached thereto to transfect OP9 cells [Nakano T., Semin.Immunol. 7(3), 197-203, 1995] with pEF-BOS-mAGF. The transfected cells were cultured in the presence of 300 µg/mL Geneticin (Roche Diagnostics) to obtain a cell line (OP9/AGF) stably expressing mouse AGF. As a negative control, an OP9 cell line (OP9/vector) transfected with the pEF-BOS-neo vector without the mouse AGF gene was used.

### Referential example 5: Activity of AGF for proliferating chondrocytes

An activity of AGF for proliferating chondrocytes was analyzed in vitro as described below. Mouse chondrogenic cell line ATDC5 [Atsumi T. et al., Cell Differ.Dev. 30(2), 109-116, 1990] was infected with an expression vector pEGFPMY [Onai N.et al., Blood, 96(6), 2074-2080, 2000] for expressing green fluorescent protein (GFP) which may be used in a retrovirus expression system. The preparation of retrovirus and the infection to ATDC5 were carried out in accordance with the Miyamoto et al. method [Miyamoto T. et al., Blood, 98(8), 2544-2554, 2001].

The infected cells were cultured in an ATDC5 culture medium [DMEM/F-12 (Lifetechnologies), 5% FCS, 5 µg/mL insulin, 5 µg/mL transferrin, and 3×10⁻⁸ mol/L sodium selenite]. Before reaching confluent conditions, cells were treated with trypsin to detach them from the culture plate. The cells were suspended and subjected to a cell sorter (FACS vantage; Becton Dickinson). ATDC5 cells having fluorescence derived from GFP were separated and collected to obtain ATDC5 cells expressing GFP. The obtained GFP-expressing ATDC5 cells were cultured and proliferated, and the above procedures (i.e., separation and collection of GFP-expressing cells by the cell sorter) were repeated to obtain ATDC5 (ATDC5/GFP) stably expressing GFP. The AGF-stably-expressing OP9 cell line (OP9/AGF) and the control OP9 cell line (OP9/vector) prepared in Referential example 4 were cultured in a 12-well plate to become confluent. The ATDC5/GFP cells (50 cells/well) were added to each well of the plate, and cultured in the ATDC5 culture medium for 14 days. The proliferation of ATDC5/GFP was analyzed using a fluorescent microscope.

As a result, when the control OP9 cell line (OP9/vector) was used as a feeder cell, no ATDC5/GFP colony was formed, and ATDC5/GFP cells were not proliferated. In contrast, when the AGF-stably-expressing OP9 cell line (OP9/AGF) was used as a feeder cell, many ATDC5/GFP colonies were formed (approximately 16 colonies per well), and ATDC5/GFP cells were remarkably proliferated. Because OP9/AGF expresses the recombinant AGF protein, it was found that the recombinant AGF exhibits an activity of proliferating ATDC5/GFP cells, i.e., the recombinant AGF exhibits an activity of proliferating chondrocytes.

### INDUSTRIAL APPLICABILITY

The AGF promoter, the screening method, and the nonhuman knockout animal according to the present invention may be used in screening an antiobesity agent, an antidiabetic agent, and/or a hypolipidemic agent. The nonhuman transgenic animal of the present invention may be used in developing an antiobesity agent, an antidiabetic agent, and/or a hypolipidemic agent.
Although the present invention has been described with reference to specific embodiments, various changes and modifications obvious to those skilled in the art are possible without departing from the scope of the appended claims.

### BRIEF DESCRIPTION OF THE DRAWINGS

[Figure 1]
   Figure 1 is a schematic drawing showing the structure of plasmid pBS-loxP-lox71-mAGF-βgeo. The abbreviations "pro." and "pri." mean a promoter and a primer, respectively.
[Figure 2]
   Figure 2 is a graph showing changes in body weight of AGF KO mice. The horizontal axis indicates an age in weeks (weeks), and the vertical axis indicates body weight (g). The abbreviations "WT", "HTR", and "HM" mean WT mice, heterozygous KO mice, and homozygous KO mice, respectively.
[Figure 3]
   Figure 3 is a graph showing changes in the weight of genital fat pads (white adipose tissue) of CAG-AGF Tg mice (normal diet). The vertical axis indicates [weight of white adipose tissue (g)]/[body weight (g)]. The abbreviations "WT" and "Tg" mean WT mice and Tg mice, respectively.
[Figure 4]
   Figure 4 is a graph showing changes in the weight of genital fat pads (white adipose tissue) of CAG-AGF Tg mice (high fat diet). The vertical axis indicates [weight of white adipose tissue (g)]/[body weight (g)]. The abbreviations "WT" and "Tg" mean WT mice and Tg mice, respectively.
[Figure 5]
   Figure 5 is a graph showing changes in the weight of genital fat pads (white adipose tissue) of AGF KO mice (weight of white adipose tissue). The vertical axis indicates the weight of white adipose tissue (g). The abbreviations "WT", "HTR", and "HM" mean WT mice, heterozygous KO mice, and homozygous KO mice, respectively.
[Figure 6]
   Figure 6 is a graph showing changes in the weight of genital fat pads (white adipose tissue) of AGF KO mice (weight of white adipose tissue/body weight). The vertical axis indicates [weight of white adipose tissue (g)]/[body weight (g)]. The abbreviations "WT", "HTR", and "HM" mean WT mice, heterozygous KO mice, and homozygous KO mice, respectively.
[Figure 7]
   Figure 7 is a microphotograph showing the form of adipocytes in the CAG-AGF Tg mouse.
[Figure 8]
   Figure 8 is a microphotograph showing the form of adipocytes in the AGF homozygous KO mouse.
[Figure 9]
   Figure 9 is a microphotograph showing the form of adipocytes in the littermate WT mouse.
[Figure 10]
   Figure 10 is a graph showing TG contents in tissue (liver) of the CAG-AGF Tg mouse. The horizontal axis indicates the period of breeding with a high fat diet (month). The vertical axis indicates a TG content (mg/g tissue). The abbreviations "WT" and "Tg" mean WT mice and Tg mice, respectively.
[Figure 11]
   Figure 11 is a graph showing TG contents in tissue (skeletal muscles) of the CAG-AGF Tg mouse. The horizontal axis indicates the period of breeding with a high fat diet (months). The vertical axis indicates a TG content (mg/g tissue). The abbreviations "WT" and "Tg" mean WT mice and Tg mice, respectively.
[Figure 12]
   Figure 12 is a graph showing TG contents in tissue of the AGF KO mice. The vertical axis indicates a TG content (mg/g tissue). The abbreviations "L" and "SM" in the horizontal axis mean the liver and skeletal muscles, respectively. The abbreviations "WT", "HTR", and "HM" mean WT mice, heterozygous KO mice, and homozygous KO mice, respectively.
[Figure 13]
   Figure 13 is a graph showing the result (blood glucose level) of glucose tolerance test in the AGF KO mice. The horizontal axis indicates a time (minutes), and the vertical axis indicates a blood glucose level (mg/dL). The abbreviations "WT" and "HM" mean WT mice and homozygous KO mice, respectively.
[Figure 14]
   Figure 14 is a graph showing the result (serum insulin) of glucose tolerance test in the AGF KO mice. The horizontal axis indicates a time (minutes), and the vertical axis indicates serum insulin (ng/mL). The abbreviations "WT" and "HM" mean WT mice and homozygous KO mice, respectively.
[Figure 15]
   Figure 15 is a graph showing oxygen consumption in the CAG-AGF Tg mice. In the horizontal axis, lane 1, lane 2, and lane 3 indicate results in the light period, the dark period, and 24 hours, respectively. The vertical axis indicates VO₂ (mL/kg/min). The abbreviations "WT" and "Tg" mean WT mice and Tg mice, respectively.
[Figure 16]
   Figure 16 is a graph showing a luciferase activity. The vertical axis indicates luciferase/β-gal.
[Figure 17]
   Figure 17 is a graph showing a luciferase activity. The vertical axis indicates luciferase/β-gal.

### FREE TEXT IN SEQUENCE LISTING

Features of "Artificial Sequence" are described in the numeric identifier <223> in the Sequence Listing. More particularly, each of the nucleotide sequences of SEQ ID NOS: 6, 7, 9, 10, 13, 14, 16, 19, 20, 45 to 53, and 56 is an artificially synthesized primer sequence. The nucleotide sequence of SEQ ID NO: 15 is a sequence containing loxP.

## Claims

1. (a) A DNA exhibiting a promoter activity for an angiopoietin-related growth factor, and consisting of a nucleotide sequence in which 1 to 10 nucleotides are substituted, deleted, added, and/or inserted in the nucleotide sequence consisting of nucleotides 2705-3001 of SEQ ID NO: 1, or
(b) a DNA exhibiting a promoter activity for an angiopoietin-related growth factor, and consisting of a nucleotide sequence having a 90% or more homology with that consisting of nucleotides 2705-3001 of SEQ ID NO: 1.

2. A DNA consisting of the nucleotide sequence consisting of nucleotides 2705-3001 of SEQ ID NO: 1.

3. A recombinant vector **characterized by** comprising the DNA according to claim 1 or 2 and exhibiting a promoter activity for an angiopoietin-related growth factor.

4. A transformant **characterized by** comprising the DNA according to claim 1 or 2 and exhibiting a promoter activity for an angiopoietin-related growth factor.

5. A method for screening an antiobesity agent, an antidiabetic agent, and/or a hypolipidemic agent, **characterized by** comprising the steps of:
i) bringing a substance to be tested into contact with the transformant according to claim 4, and
ii) measuring a promoter activity for an angiopoietin-related growth factor and analyzing a test substance dependent change in the promoter activity.

6. The screening method according to claim 5, wherein the transformant contains a reporter gene located downstream of the DNA according to claim 1 or 2, and the promoter activity for an angiopoietin-related growth factor is measured by analyzing an expression of the reporter gene.

7. A nonhuman knockout animal **characterized in that** a polynucleotide encoding an angiopoietin-related growth factor is functionally deficient on a chromosome.

8. A nonhuman transgenic animal which is a nonhuman animal or an offspring animal thereof obtained by ontogenesis from totipotent cells in which a polynucleotide is introduced together with a CAG promoter, wherein the polynucleotide is carried on a chromosome, a polypeptide encoded by the polynucleotide is expressed in a somatic cell, and the polypeptide is selected from the group consisting of:
(a) a polypeptide exhibiting an activity of suppressing an increase in body weight, and comprising an amino acid sequence consisting of amino acids 1-450 of SEQ ID NO: 3 or amino acids 1-433 of SEQ ID NO: 5,
(b) a polypeptide exhibiting an activity of suppressing an increase in body weight, and comprising an amino acid sequence in which 1 to 10 amino acids are substituted, deleted, and/or inserted in an amino acid sequence consisting of amino acids 1-450 of SEQ ID NO: 3 or amino acids 1-433 of SEQ ID NO: 5,
(c) a polypeptide exhibiting an activity of suppressing an increase in body weight, and encoded by a DNA which hybridizes under stringent conditions to a DNA encoding an amino acid sequence consisting of amino acids 1-450 of SEQ ID NO: 3 or amino acids 1-433 of SEQ ID NO: 5, and
(d) a polypeptide exhibiting an activity of suppressing an increase in body weight, and comprising an amino acid sequence having a 95% or more homology with that consisting of amino acids 1-450 of SEQ ID NO: 3 or amino acids 1-433 of SEQ ID NO: 5.


SEQUENCE LISTING

<110> Yamanouchi Pharmaceutical Co., Ltd.
<110> Keio University

<120> Method of screening antiobesity agents and animal model of obesity

<130> YK0417PCT711

<150> JP 2003-163016
<151> 2003-06-06

<150> JP 2004-111500
<151> 2004-04-05

<160> 59

<170> PatentIn version 3.1

<210> 1
<211> 3028
<212> DNA
<213> Homo sapiens

<220>
<223> Inventor: Yasunaga, Kunio; Yamaji, Noboru; Suda, Toshio
      Inventor: Oike, Yuichi

<400> 1
gtcttggcct caggcctctg gcttcttccc caaaaccttg cagtggggga aagggacacc 60

ccctcactgg gctgaacatt actctctcct cccccatcgg cctcctctcc ctccttgctc 120

tctctggcac cccaattctt cctcctctcc cccagatgtg tctcccctcc ccctcgtctt 180

ccccctgtct ctttctccct cttgtaactt tgtctctgtc atcagttgtc tctttcatca 240

tttataactg aagatggaaa ctaaagaagt ttcctttctc tgtgtctggc cccatccctg 300

tttctttctt ctcatctgcc tccctgtggt ctctccccat cttcccatct ctctctgtct 360

ctccccatct ccccgtctct ccctttctct gccatctccc tatgtctctc ccatctccct 420

atgcctctcc catctccatc tgtctgactc tctgtattct tcagcccctc tctgtgactt 480

atgaagttcc ctccctctct actgccaatg gggaagccag agggggctga gggtgggggg 540

ccgaggggcc cagtgtgggg gccccaaccc aggcccagag cccagcctgg cagcctctct 600

cagatctggg acgaccattt cctttttcaa ccctggagtc cactcgagtt actcgggagc 660

tcaggatggt gcagaccagg ggaaggaggt gactctcccg gcgtgtggct ggtcgtcggt 720

ggggcctttg gacaggtctc agaccctggg cctgggctct gtggggctct atgtgagttt 780

ctttgtaccc ctgggaaatg actgcatggt tacttccccc agttccgtcc tcgtgtgatt 840

tgacttgtgt gaaaatgtga ccgagtgtgt gggagacaga cggtgacctc tgtggtgcgg 900

tggataggtt tccgggtgtc aactgtgtgt acctgttttg tatctgtgca ctactgtgtg 960

atcacaagaa ccaggatgtg gacaagcctg acacacagat aaagacccac tcagcccacc 1020

ccatttccct ttaagttgtg atgtgtgtgt gaccatattt ggaacataca gttgttgcct 1080

gaaactgtta tgtttccagc tgtgtctttt taaaaatttt ttttaatcaa tgagatatcc 1140

tttatttatg aaagcatagt tccaatgcag aagtcaagct ttatggttat atattaggat 1200

aggttcagat ccaatatgac aatgtttagc actaacttta acatgttaaa gctctgattc 1260

ctgaatgttt ttctcactta atatattctc tccaatgaca agctggtgtg cttggcttct 1320

tatgctgaaa aatatatgcc tgaattctat aacaagctat taacaaaatc atgaatagtt 1380

aattaaataa gtgccaacac tacatggaac ttactggcca ccaactcaga accataaaac 1440

tacacacaca cacacacaca cacacacaca ccacacacac aaacaaaaca aaaaaaacac 1500

caaagaaaga tagctatcac tgtacatatg aatactgtcc aggatatcag atcaattatc 1560

aaatccaagg tctctggtgt tttgtctcac agtgttgaat atatgaatgc ttctctatta 1620

tactgacttg tattattttc aacaaataat tatgtggact caaaatctta catttttatt 1680

tcttttcttt tttttttttt ttttttgaga cagagtctta ctctgtcgcc caggctggag 1740

tgcagtggct cactgcaacc tccgcccccc gggttcaagc gattctcctg cctcagcctc 1800

ctgagtagct gggattacag gcatgggcca ccacacccgg caaatttttg tatttttagt 1860

agagacgggg tttcaccatc ttggccaggc tggtctcgaa ttgctgacct cgtgatctgc 1920

ccgccttggc ctcccaaagt gctgggatta caggcgtgag ccaccgcgcc cagccttaca 1980

tttttatttc taattataaa atccagatta atccaaaaaa cataaatcac atgtaactga 2040
ggtagggttt tcactttaag aatatattgt ttctatcaca atttaaaaat ttacagctgt 2100
gtcttttata tttatttttt attttaatta attaattcct cactttatta ttatttattt 2160
attatttatt tatttattta tttatttatt tttgagatgg aatttcagtc ttgtcgccca 2220
ggctggagtg catgcggttg ctcgatctcg gcttactgca acctccacct cctggcttca 2280
agcaattctc ctgcctcagc ctcccgagta gctgggatta ctggcgcatg ccaccatgcc 2340
cagctaattt ttgtattgtt agtagagacg gggttttagc atgttggcaa ggatggtttc 2400
gaactcctga cctcaggtaa tccacctgcc ccggcctccc aaagtgctgg ctttcaggca 2460
tgagccactg cacccagact ttcgctgggt gttaatgtgt gcgtgcgtgc aaatgtgtgt 2520
gtgtgtgttt gtgtccactc tgttatgtgc ccatatgtgt gtgacagctg tggtgtacat 2580
gtgtgagttt ctgagtcaag ctaccttggc tggctcccat cctggtctct ttttggctgt 2640
gtgaccgcag gcaagttgct taccctctct gagcctcagt tttccctgct gtaaaatgag 2700
ggtacataca gaatccagct caggttgtaa ggatcaaata cgtaaaaggt gtggagagtt 2760
tagagccaag ccctacacac ggtaagtccc cagtgatgtt cctctttcta tgtgtggtgt 2820
gtgcgtgtct ggggctggga tcacgcctgg ctgccccagg gcctgcagcc tcccacgctc 2880
tgggctctgc agtggagggt gtgggggaaa gggagggatg tccaggacac tgacctgtgt 2940
tatcaggctc tggtggcctg ggcagggggg tctggcaatt ctgtctcatt tctcattcgg 3000
gaaatgctaa atatttatag tttgggct 3028

<210> 2
<211> 1413
<212> DNA
<213> Homo sapiens

<220>
<221> CDS
<222> (1)..(1413)
<223>

<220>
<221> mat_peptide
<222> (61)..(1410)
<223>

<400> 2
atg ggg aag ccc tgg ctg cgt gcg cta cag ctg ctg ctc ctg ctg ggc 48
Met Gly Lys Pro Trp Leu Arg Ala Leu Gln Leu Leu Leu Leu Leu Gly
-20 -15 -10 -5

gcg tcg tgg gcg cgg gcg ggc gcc ccg cgc tgc acc tac acc ttc gtg 96
Ala Ser Trp Ala Arg Ala Gly Ala Pro Arg Cys Thr Tyr Thr Phe Val
-1 1 5 10

ctg ccc ccg cag aag ttc acg ggc gct gtg tgc tgg agc ggc ccc gca 144
Leu Pro Pro Gln Lys Phe Thr Gly Ala Val Cys Trp Ser Gly Pro Ala
15 20 25

tcc acg cgg gcg acg ccc gag gcc gcc aac gcc agc gag ctg gcg gcg 192
Ser Thr Arg Ala Thr Pro Glu Ala Ala Asn Ala Ser Glu Leu Ala Ala
30 35 40

ctg cgc atg cgc gtc ggc cgc cac gag gag ctg tta cgc gag ctg cag 240
Leu Arg Met Arg Val Gly Arg His Glu Glu Leu Leu Arg Glu Leu Gln
45 50 55 60

agg ctg gcg gcg gcc gac ggc gcc gtg gcc ggc gag gtg cgc gcg ctg 288
Arg Leu Ala Ala Ala Asp Gly Ala Val Ala Gly Glu Val Arg Ala Leu
65 70 75

cgc aag gag agc cgc ggc ctg agc gcg cgc ctg ggc cag ttg cgc gcg 336
Arg Lys Glu Ser Arg Gly Leu Ser Ala Arg Leu Gly Gln Leu Arg Ala
80 85 90

cag ctg cag cac gag gcg ggg ccc ggg gcg ggc ccg ggg gcg gat ctg 384
Gln Leu Gln His Glu Ala Gly Pro Gly Ala Gly Pro Gly Ala Asp Leu
95 100 105

ggg gcg gag cct gcc gcg gcg ctg gcg ctg ctc ggg gag cgc gtg ctc 432
Gly Ala Glu Pro Ala Ala Ala Leu Ala Leu Leu Gly Glu Arg Val Leu
110 115 120

aac gcg tcc gcc gag gct cag cgc gca gcc gcc cgg ttc cac cag ctg 480
Asn Ala Ser Ala Glu Ala Gln Arg Ala Ala Ala Arg Phe His Gln Leu
125 130 135 140

gac gtc aag ttc cgc gag ctg gcg cag ctc gtc acc cag cag agc agt 528

Asp Val Lys Phe Arg Glu Leu Ala Gln Leu Val Thr Gln Gln Ser Ser
145 150 155

ctc atc gcc cgc ctg gag cgc ctg tgc ccg gga ggc gcg ggc ggg cag 576
Leu Ile Ala Arg Leu Glu Arg Leu Cys Pro Gly Gly Ala Gly Gly Gln
160 165 170

cag cag gtc ctg ccg cca ccc cca ctg gtg cct gtg gtt ccg gtc cgt 624
Gln Gln Val Leu Pro Pro Pro Pro Leu Val Pro Val Val Pro Val Arg
175 180 185

ctt gtg ggt agc acc agt gac acc agt agg atg ctg gac cca gcc cca 672
Leu Val Gly Ser Thr Ser Asp Thr Ser Arg Met Leu Asp Pro Ala Pro
190 195 200

gag ccc cag aga gac cag acc cag aga cag cag gag ccc atg gct tct 720
Glu Pro Gln Arg Asp Gln Thr Gln Arg Gln Gln Glu Pro Met Ala Ser
205 210 215 220

ccc atg cct gca ggt cac cct gcg gtc ccc acc aag cct gtg ggc ccg 768
Pro Met Pro Ala Gly His Pro Ala Val Pro Thr Lys Pro Val Gly Pro
225 230 235

tgg cag gat tgt gca gag gcc cgc cag gca ggc cat gaa cag agt gga 816
Trp Gln Asp Cys Ala Glu Ala Arg Gln Ala Gly His Glu Gln Ser Gly
240 245 250

gtg tat gaa ctg cga gtg ggc cgt cac gta gtg tca gta tgg tgt gag 864
Val Tyr Glu Leu Arg Val Gly Arg His Val Val Ser Val Trp Cys Glu
255 260 265

cag caa ctg gag ggt gga ggc tgg act gtg atc cag cgg agg caa gat 912
Gln Gln Leu Glu Gly Gly Gly Trp Thr Val Ile Gln Arg Arg Gln Asp
270 275 280

ggt tca gtc aac ttc ttc act acc tgg cag cac tat aag gcg ggc ttt 960
Gly Ser Val Asn Phe Phe Thr Thr Trp Gln His Tyr Lys Ala Gly Phe
285 290 295 300

ggg cgg cca gac gga gaa tac tgg ctg ggc ctt gaa ccc gtg tat cag 1008
Gly Arg Pro Asp Gly Glu Tyr Trp Leu Gly Leu Glu Pro Val Tyr Gln
305 310 315

ctg acc agc cgt ggg gac cat gag ctg ctg gtt ctc ctg gag gac tgg 1056
Leu Thr Ser Arg Gly Asp His Glu Leu Leu Val Leu Leu Glu Asp Trp
320 325 330

ggg ggc cgt gga gca cgt gcc cac tat gat ggc ttc tcc ctg gaa ccc 1104

Gly Gly Arg Gly Ala Arg Ala His Tyr Asp Gly Phe Ser Leu Glu Pro
335 340 345

gag agc gac cac tac cgc ctg cgg ctt ggc cag tac cat ggt gat gct 1152
Glu Ser Asp His Tyr Arg Leu Arg Leu Gly Gln Tyr His Gly Asp Ala
350 355 360

gga gac tct ctt tcc tgg cac aat gac aag ccc ttc agc acc gtg gat 1200
Gly Asp Ser Leu Ser Trp His Asn Asp Lys Pro Phe Ser Thr Val Asp
365 370 375 380

agg gac cga gac tcc tat tct ggt aac tgt gcc ctg tac cag cgg gga 1248
Arg Asp Arg Asp Ser Tyr Ser Gly Asn Cys Ala Leu Tyr Gln Arg Gly
385 390 395

ggc tgg tgg tac cat gcc tgt gcc cac tcc aac ctc aac ggt gtg tgg 1296
Gly Trp Trp Tyr His Ala Cys Ala His Ser Asn Leu Asn Gly Val Trp
400 405 410

cac cac ggc ggc cac tac cga agc cgc tac cag gat ggt gtc tac tgg 1344
His His Gly Gly His Tyr Arg Ser Arg Tyr Gln Asp Gly Val Tyr Trp
415 420 425

gct gag ttt cgt ggt ggg gca tat tct ctc agg aag gcc gcc atg ctc 1392
Ala Glu Phe Arg Gly Gly Ala Tyr Ser Leu Arg Lys Ala Ala Met Leu
430 435 440

att cgg ccc ctg aag ctg tga 1413
Ile Arg Pro Leu Lys Leu
445 450

<210> 3
<211> 470
<212> PRT
<213> Homo sapiens

<400> 3

Met Gly Lys Pro Trp Leu Arg Ala Leu Gln Leu Leu Leu Leu Leu Gly
-20 -15 -10 -5

Ala Ser Trp Ala Arg Ala Gly Ala Pro Arg Cys Thr Tyr Thr Phe Val
-1 1 5 10

Leu Pro Pro Gln Lys Phe Thr Gly Ala Val Cys Trp Ser Gly Pro Ala

15 20 25

Ser Thr Arg Ala Thr Pro Glu Ala Ala Asn Ala Ser Glu Leu Ala Ala
30 35 40

Leu Arg Met Arg Val Gly Arg His Glu Glu Leu Leu Arg Glu Leu Gln
45 50 55 60

Arg Leu Ala Ala Ala Asp Gly Ala Val Ala Gly Glu Val Arg Ala Leu
65 70 75

Arg Lys Glu Ser Arg Gly Leu Ser Ala Arg Leu Gly Gln Leu Arg Ala
80 85 90

Gln Leu Gln His Glu Ala Gly Pro Gly Ala Gly Pro Gly Ala Asp Leu
95 100 105

Gly Ala Glu Pro Ala Ala Ala Leu Ala Leu Leu Gly Glu Arg Val Leu
110 115 120

Asn Ala Ser Ala Glu Ala Gln Arg Ala Ala Ala Arg Phe His Gln Leu
125 130 135 140

Asp Val Lys Phe Arg Glu Leu Ala Gln Leu Val Thr Gln Gln Ser Ser
145 150 155

Leu Ile Ala Arg Leu Glu Arg Leu Cys Pro Gly Gly Ala Gly Gly Gln
160 165 170

Gln Gln Val Leu Pro Pro Pro Pro Leu Val Pro Val Val Pro Val Arg
175 180 185

Leu Val Gly Ser Thr Ser Asp Thr Ser Arg Met Leu Asp Pro Ala Pro
190 195 200

Glu Pro Gln Arg Asp Gln Thr Gln Arg Gln Gln Glu Pro Met Ala Ser

205                 210                 215                 220

Pro Met Pro Ala Gly His Pro Ala Val Pro Thr Lys Pro Val Gly Pro
                225                 230                 235

Trp Gln Asp Cys Ala Glu Ala Arg Gln Ala Gly His Glu Gln Ser Gly
            240                 245                 250

Val Tyr Glu Leu Arg Val Gly Arg His Val Val Ser Val Trp Cys Glu
        255                 260                 265

Gln Gln Leu Glu Gly Gly Gly Trp Thr Val Ile Gln Arg Arg Gln Asp
    270                 275                 280

Gly Ser Val Asn Phe Phe Thr Thr Trp Gln His Tyr Lys Ala Gly Phe
285                 290                 295                 300

Gly Arg Pro Asp Gly Glu Tyr Trp Leu Gly Leu Glu Pro Val Tyr Gln
                305                 310                 315

Leu Thr Ser Arg Gly Asp His Glu Leu Leu Val Leu Leu Glu Asp Trp
            320                 325                 330

Gly Gly Arg Gly Ala Arg Ala His Tyr Asp Gly Phe Ser Leu Glu Pro
        335                 340                 345

Glu Ser Asp His Tyr Arg Leu Arg Leu Gly Gln Tyr His Gly Asp Ala
    350                 355                 360

Gly Asp Ser Leu Ser Trp His Asn Asp Lys Pro Phe Ser Thr Val Asp
365                 370                 375                 380

Arg Asp Arg Asp Ser Tyr Ser Gly Asn Cys Ala Leu Tyr Gln Arg Gly
                385                 390                 395

Gly Trp Trp Tyr His Ala Cys Ala His Ser Asn Leu Asn Gly Val Trp

400                    405                    410

His His Gly Gly His Tyr Arg Ser Arg Tyr Gln Asp Gly Val Tyr Trp
        415                    420                    425

Ala Glu Phe Arg Gly Gly Ala Tyr Ser Leu Arg Lys Ala Ala Met Leu
    430                    435                    440

Ile Arg Pro Leu Lys Leu
445                    450

<210>  4
<211>  1374
<212>  DNA
<213>  Mus musculus

<220>
<221>  CDS
<222>  (1)..(1374)
<223>

<220>
<221>  mat_peptide
<222>  (73)..(1371)
<223>

<400>  4
atg ggg acc gcc agg cta cgc aag ctg caa ctg ctg ctt ctg ctg ggc        48
Met Gly Thr Ala Arg Leu Arg Lys Leu Gln Leu Leu Leu Leu Leu Gly
                -20                    -15                    -10

gct tgg agg gcg ctc gga ggt gcc gcg cgt tgc cgc gtc acc cta gtt        96
Ala Trp Arg Ala Leu Gly Gly Ala Ala Arg Cys Arg Val Thr Leu Val
            -5                  -1  1                   5

ttg tcc ccg cag aag gca act agc gcc gtc tgc agg agc tca gag gcc       144
Leu Ser Pro Gln Lys Ala Thr Ser Ala Val Cys Arg Ser Ser Glu Ala
    10                  15                  20

acc caa gac agc gaa ctg gcc acg ctg cgc atg cgc ctg ggt cgc cac       192
Thr Gln Asp Ser Glu Leu Ala Thr Leu Arg Met Arg Leu Gly Arg His
25                  30                  35                  40

gag gag ctg ctg cgc gcg ctg caa agg cgt gcg gcg gag ggt ggt gcg 240
Glu Glu Leu Leu Arg Ala Leu Gln Arg Arg Ala Ala Glu Gly Gly Ala
45 50 55

ctc gcg gac gag gtg cgc gca ctg cgc gag cac agt ctc acc ctg aac 288
Leu Ala Asp Glu Val Arg Ala Leu Arg Glu His Ser Leu Thr Leu Asn
60 65 70

acg cgc ctg ggc cag ctg cgc gcg caa ttg cag cag gag gcg agg gcg 336
Thr Arg Leu Gly Gln Leu Arg Ala Gln Leu Gln Gln Glu Ala Arg Ala
75 80 85

gag cct gac ctg ggg gcg gag cct gct gct gca ctt ggt ttg cta gcc 384
Glu Pro Asp Leu Gly Ala Glu Pro Ala Ala Ala Leu Gly Leu Leu Ala
90 95 100

gag cgc gcg ctg gac gct gag gcc gaa gcg cgc cgg acg acg gca cgc 432
Glu Arg Ala Leu Asp Ala Glu Ala Glu Ala Arg Arg Thr Thr Ala Arg
105 110 115 120

ctg cag cag ctg gac gca cag ctc cgt gag cat gcg cag ctc atg agc 480
Leu Gln Gln Leu Asp Ala Gln Leu Arg Glu His Ala Gln Leu Met Ser
125 130 135

cag cat agc agc ctc ctc ggc cgc ctg caa cgc gcg tgc gcg ggc ccg 528
Gln His Ser Ser Leu Leu Gly Arg Leu Gln Arg Ala Cys Ala Gly Pro
140 145 150

gaa cgg gga cag cag cag gtc ctg cca ctg ccc ctg gcg cct ctg gtg 576
Glu Arg Gly Gln Gln Gln Val Leu Pro Leu Pro Leu Ala Pro Leu Val
155 160 165

cct ctg agc ctc gtg ggc agt gcc agc aac acc agc agg agg ctg gac 624
Pro Leu Ser Leu Val Gly Ser Ala Ser Asn Thr Ser Arg Arg Leu Asp
170 175 180

caa act cca gag cac cag aga gag cag agc ttg aga cag cag ggg cct 672
Gln Thr Pro Glu His Gln Arg Glu Gln Ser Leu Arg Gln Gln Gly Pro
185 190 195 200

cca tct tct ctg ctg ccc aca ggg cac ctt gct gtc ccc aca agg cca 720
Pro Ser Ser Leu Leu Pro Thr Gly His Leu Ala Val Pro Thr Arg Pro
205 210 215

gtg ggc cca tgg agg gat tgt gca gag gct cac ggg gca ggt cac tgg 768
Val Gly Pro Trp Arg Asp Cys Ala Glu Ala His Gly Ala Gly His Trp
220 225 230

cag agt gga gtg tat gac ctg cgg ctg ggc cgt cgt gta gta gcc gtg 816
Gln Ser Gly Val Tyr Asp Leu Arg Leu Gly Arg Arg Val Val Ala Val
235 240 245

tgg tgt gaa cag cag cag gaa ggt gga ggc tgg act gtc atc cag aga 864
Trp Cys Glu Gln Gln Gln Glu Gly Gly Gly Trp Thr Val Ile Gln Arg
250 255 260

cgg cag gac ggc tct gtc aac ttc ttc acc aac tgg cag cac tac aag 912
Arg Gln Asp Gly Ser Val Asn Phe Phe Thr Asn Trp Gln His Tyr Lys
265 270 275 280

gcg ggc ttt ggg cgt cca gaa gga gaa tac tgg ctg ggc ctg gaa cct 960
Ala Gly Phe Gly Arg Pro Glu Gly Glu Tyr Trp Leu Gly Leu Glu Pro
285 290 295

gtg cat cag gtg aca agc cgt ggg gac cac gag ctg ctg ata ctc cta 1008
Val His Gln Val Thr Ser Arg Gly Asp His Glu Leu Leu Ile Leu Leu
300 305 310

gag gac tgg ggg ggc cgt gca gca cgc gcc cac tac gac agc ttc tcc 1056
Glu Asp Trp Gly Gly Arg Ala Ala Arg Ala His Tyr Asp Ser Phe Ser
315 320 325

ttg gag cct gag agt gac cac tac cgt ctg cgg ctt ggc cag tac cac 1104
Leu Glu Pro Glu Ser Asp His Tyr Arg Leu Arg Leu Gly Gln Tyr His
330 335 340

ggc gat gcc gga gac tcc ctc tct tgg cac aat gac aaa cct ttc agc 1152
Gly Asp Ala Gly Asp Ser Leu Ser Trp His Asn Asp Lys Pro Phe Ser
345 350 355 360

act gtg gat agg gac aga gac tca tat tct ggt aac tgt gcc ctg tac 1200
Thr Val Asp Arg Asp Arg Asp Ser Tyr Ser Gly Asn Cys Ala Leu Tyr
365 370 375

cat cgt ggg ggc tgg tgg tac cat gcc tgt gcc cac tct aac ctc aat 1248
His Arg Gly Gly Trp Trp Tyr His Ala Cys Ala His Ser Asn Leu Asn
380 385 390

gga gta tgg tat cat gga ggt cat tac cgg agc cga tac cag gac ggg 1296
Gly Val Trp Tyr His Gly Gly His Tyr Arg Ser Arg Tyr Gln Asp Gly
395 400 405

gtc tac tgg gcc gag ttc cgt ggt ggg gcg tac tct ctg aag aaa gct 1344
Val Tyr Trp Ala Glu Phe Arg Gly Gly Ala Tyr Ser Leu Lys Lys Ala
410 415 420

gtt atg ttg acc cgg ctt gtg cgc ttg tga                                    1374
Val Met Leu Thr Arg Leu Val Arg Leu
425                 430

<210>  5
<211>  457
<212>  PRT
<213>  Mus musculus

<400>  5

Met Gly Thr Ala Arg Leu Arg Lys Leu Gln Leu Leu Leu Leu Leu Gly
                -20                 -15                 -10

Ala Trp Arg Ala Leu Gly Gly Ala Ala Arg Cys Arg Val Thr Leu Val
            -5                  -1  1               5

Leu Ser Pro Gln Lys Ala Thr Ser Ala Val Cys Arg Ser Ser Glu Ala
    10                  15                  20

Thr Gln Asp Ser Glu Leu Ala Thr Leu Arg Met Arg Leu Gly Arg His
25                  30                  35                  40

Glu Glu Leu Leu Arg Ala Leu Gln Arg Arg Ala Ala Glu Gly Gly Ala
                45                  50                  55

Leu Ala Asp Glu Val Arg Ala Leu Arg Glu His Ser Leu Thr Leu Asn
            60                  65                  70

Thr Arg Leu Gly Gln Leu Arg Ala Gln Leu Gln Gln Glu Ala Arg Ala
        75                  80                  85

Glu Pro Asp Leu Gly Ala Glu Pro Ala Ala Ala Leu Gly Leu Leu Ala
    90                  95                  100

Glu Arg Ala Leu Asp Ala Glu Ala Glu Ala Arg Arg Thr Thr Ala Arg
105                 110                 115                 120

Leu Gln Gln Leu Asp Ala Gln Leu Arg Glu His Ala Gln Leu Met Ser
                125                 130                 135

Gln His Ser Ser Leu Leu Gly Arg Leu Gln Arg Ala Cys Ala Gly Pro
            140                 145                 150

Glu Arg Gly Gln Gln Gln Val Leu Pro Leu Pro Leu Ala Pro Leu Val
        155                 160                 165

Pro Leu Ser Leu Val Gly Ser Ala Ser Asn Thr Ser Arg Arg Leu Asp
    170                 175                 180

Gln Thr Pro Glu His Gln Arg Glu Gln Ser Leu Arg Gln Gln Gly Pro
185                 190                 195                 200

Pro Ser Ser Leu Leu Pro Thr Gly His Leu Ala Val Pro Thr Arg Pro
                205                 210                 215

Val Gly Pro Trp Arg Asp Cys Ala Glu Ala His Gly Ala Gly His Trp
            220                 225                 230

Gln Ser Gly Val Tyr Asp Leu Arg Leu Gly Arg Arg Val Val Ala Val
        235                 240                 245

Trp Cys Glu Gln Gln Gln Glu Gly Gly Gly Trp Thr Val Ile Gln Arg
    250                 255                 260

Arg Gln Asp Gly Ser Val Asn Phe Phe Thr Asn Trp Gln His Tyr Lys
265                 270                 275                 280

Ala Gly Phe Gly Arg Pro Glu Gly Glu Tyr Trp Leu Gly Leu Glu Pro
                285                 290                 295

Val His Gln Val Thr Ser Arg Gly Asp His Glu Leu Leu Ile Leu Leu
            300                 305                 310

Glu Asp Trp Gly Gly Arg Ala Ala Arg Ala His Tyr Asp Ser Phe Ser
        315                 320                 325

Leu Glu Pro Glu Ser Asp His Tyr Arg Leu Arg Leu Gly Gln Tyr His
    330                 335                 340

Gly Asp Ala Gly Asp Ser Leu Ser Trp His Asn Asp Lys Pro Phe Ser
345                 350                 355                 360

Thr Val Asp Arg Asp Arg Asp Ser Tyr Ser Gly Asn Cys Ala Leu Tyr
                365                 370                 375

His Arg Gly Gly Trp Trp Tyr His Ala Cys Ala His Ser Asn Leu Asn
            380                 385                 390

Gly Val Trp Tyr His Gly Gly His Tyr Arg Ser Arg Tyr Gln Asp Gly
        395                 400                 405

Val Tyr Trp Ala Glu Phe Arg Gly Gly Ala Tyr Ser Leu Lys Lys Ala
    410                 415                 420

Val Met Leu Thr Arg Leu Val Arg Leu
425                 430

<210>  6
<211>  28
<212>  DNA
<213>  Artificial Sequence

<220>
<223>  Description of Artificial Sequence: an artificially synthesized
       primer sequence

<400>  6
ctagactagt tgcaaaggcg tgcggcgg                                        28

<210>  7
<211>  36

<212>  DNA
<213>  Artificial Sequence

<220>
<223>  Description of Artificial Sequence: an artificially synthesized
       primer sequence

<400>  7
ctagactagt ggatccgcag gcttgctttg acttac                                36


<210>  8
<211>  536
<212>  DNA
<213>  Mus musculus

<400>  8
gcccatggag ggattgtgca gaggctcacg gggcaggtca ctggcagagt ggagtgtatg      60

acctgcggct gggccgtcgt gtagtagccg tgtggtgtga acagcagcag gaagtggagg     120

ctggactgtc atccagagac ggcaggacgg ctctgtcaac ttcttcacca actggcagca     180

ctacaaggtg tgtgcttgtg gtgggggtgt cagagactgc tgggcagaga ggacgccccc     240

accctcttcc tcctaccctt ccaggcgggc tttgggcgtc cagaaggaga atactggctg     300

ggcctggaac ctgtgcatca ggtgacaagc cgtggggacc acgagctgct gatactccta     360

gaggactggg ggggccgtgc agcacgcgcc cactacgaca gcttctcctt ggagcctgag     420

agtgaccact accgtctgcg gcttggccag taccacggcg atgccggaga ctccctctct     480

tggcacaatg acaaaacctt tcagcactgt ggatagggac agagactcat attctg         536


<210>  9
<211>  20
<212>  DNA
<213>  Artificial Sequence

<220>
<223>  Description of Artificial Sequence: an artificially synthesized
       primer sequence

<400>  9
agaggctatt cggctatgac                                                  20

<210>  10
<211>  20
<212>  DNA
<213>  Artificial Sequence

<220>
<223>  Description of Artificial Sequence: an artificially synthesized
       primer sequence

<400>  10
caccatgata ttcggcaagc                                                    20


<210>  11
<211>  20
<212>  DNA
<213>  Mus musculus

<400>  11
tggcctctgt tatcatgctc                                                    20


<210>  12
<211>  20
<212>  DNA
<213>  Mus musculus

<400>  12
ctacctacat ccactcctac                                                    20


<210>  13
<211>  31
<212>  DNA
<213>  Artificial Sequence

<220>
<223>  Description of Artificial Sequence: an artificially synthesized
       primer sequence

<400>  13
agaagcttca ccatggggac cgccaggcta c                                       31


<210>  14
<211>  39
<212>  DNA

<213>  Artificial Sequence

<220>
<223>  Description of Artificial Sequence: an artificially synthesized
       primer sequence

<400>  14
ccgtcgacat tagatcttca caagcgcacaag ccgggtc                              39


<210>  15
<211>  81
<212>  DNA
<213>  Artificial Sequence

<220>
<223>  Description of Artificial Sequence: Sequence containing loxP

<400>  15
gatccggaac ccttaatata acttcgtata atgtatgcta tacgaagtta ttaggtccct      60

cgacctgcag cccgggggat c                                                 81


<210>  16
<211>  20
<212>  DNA
<213>  Artificial Sequence

<220>
<223>  Description of Artificial Sequence: an artificially synthesized
       primer sequence

<400>  16
aattaaccct cactaaaggg                                                   20


<210>  17
<211>  20
<212>  DNA
<213>  Mus musculus

<400>  17
cccactacga cagcttctcc                                                   20


<210>  18
<211>  20

<212>  DNA
<213>  Mus musculus

<400>  18
agccgggtca acataacagc                                                    20


<210>  19
<211>  19
<212>  DNA
<213>  Artificial Sequence

<220>
<223>  Description of Artificial Sequence: an artificially synthesized
       primer sequence

<400>  19
gcgttaccca acttaatcg                                                     19


<210>  20
<211>  18
<212>  DNA
<213>  Artificial Sequence

<220>
<223>  Description of Artificial Sequence: an artificially synthesized
       primer sequence

<400>  20
tgtgagcgag taacaacc                                                      18


<210>  21
<211>  22
<212>  DNA
<213>  Mus musculus

<400>  21
tcgtgtagta gccgtgtggt gt                                                 22


<210>  22
<211>  20
<212>  DNA
<213>  Mus musculus

<400>  22

cacctgatgc acaggttcca                                                    20

<210>  23
<211>  19
<212>  DNA
<213>  Mus musculus

<400>  23
tggttgatcc tgccagtag                                                     19

<210>  24
<211>  20
<212>  DNA
<213>  Mus musculus

<400>  24
cgaccaaagg aaccataact                                                    20

<210>  25
<211>  23
<212>  DNA
<213>  Mus musculus

<400>  25
ccggtacagt gaaactgcga atg                                                23

<210>  26
<211>  19
<212>  DNA
<213>  Mus musculus

<400>  26
acagaaggat tgccgaaac                                                     19

<210>  27
<211>  20
<212>  DNA
<213>  Mus musculus

<400>  27
agctgatttg cctctgaatg                                                    20

<210>  28
<211>  17
<212>  DNA
<213>  Mus musculus

<400>  28
cagcggtctg cctgcgg                                                    17


<210>  29
<211>  19
<212>  DNA
<213>  Mus musculus

<400>  29
catggttgga cttcagccc                                                  19


<210>  30
<211>  17
<212>  DNA
<213>  Mus musculus

<400>  30
ggcccccagg aacttca                                                    17


<210>  31
<211>  22
<212>  DNA
<213>  Mus musculus

<400>  31
ccgaagtgcc tcccacaacg gt                                              22


<210>  32
<211>  21
<212>  DNA
<213>  Mus musculus

<400>  32
acgatgctgt cctccttgat g                                               21


<210>  33
<211>  21
<212>  DNA

<213>  Mus musculus

<400>  33
gtgtgataaa gccattgccg t                                                21

<210>  34
<211>  23
<212>  DNA
<213>  Mus musculus

<400>  34
acaaagacgg gatgctgatc gcg                                              23

<210>  35
<211>  21
<212>  DNA
<213>  Mus musculus

<400>  35
gaccctcctc aagtatggcg t                                                21

<210>  36
<211>  21
<212>  DNA
<213>  Mus musculus

<400>  36
gtctttgttg acgatggagg c                                                21

<210>  37
<211>  23
<212>  DNA
<213>  Mus musculus

<400>  37
cacgaggcca tctttgccat gct                                              23

<210>  38
<211>  22
<212>  DNA
<213>  Mus musculus

<400>  38

tgagagggaa atcgtgcgtg ac                                                22

<210>  39
<211>  22
<212>  DNA
<213>  Mus musculus

<400>  39
aagaaggaag gctggaaaag ag                                                22

<210>  40
<211>  18
<212>  DNA
<213>  Mus musculus

<400>  40
gatggcacgc agccctat                                                     18

<210>  41
<211>  22
<212>  DNA
<213>  Mus musculus

<400>  41
cgacacggag agttaaagga ag                                                22

<210>  42
<211>  26
<212>  DNA
<213>  Mus musculus

<400>  42
cattgttcga tgtgtcgcct tcttgc                                            26

<210>  43
<211>  19
<212>  DNA
<213>  Mus musculus

<400>  43
cggagcgcaa tatgaaggt                                                    19

<210> 44
<211> 20
<212> DNA
<213> Mus musculus

<400> 44
caaaaggaag acgacggagc                                                    20


<210> 45
<211> 29
<212> DNA
<213> Artificial Sequence

<220>
<223> Description of Artificial Sequence: an artificially synthesized
      primer sequence

<400> 45
aaggtaccag tgatgttcct ctttctatg                                          29


<210> 46
<211> 30
<212> DNA
<213> Artificial Sequence

<220>
<223> Description of Artificial Sequence: an artificially synthesized
      primer sequence

<400> 46
tgctagcccg aatgagaaat gagacagaat                                         30


<210> 47
<211> 30
<212> DNA
<213> Artificial Sequence

<220>
<223> Description of Artificial Sequence: an artificially synthesized
      primer sequence

<400> 47
taggtaccat acagaatcca gctcaggttg                                         30

<210> 48
<211> 28
<212> DNA
<213> Artificial Sequence

<220>
<223> Description of Artificial Sequence: an artificially synthesized
primer sequence

<400> 48
ttggtacctt ggctggctcc catcctgg 28


<210> 49
<211> 28
<212> DNA
<213> Artificial Sequence

<220>
<223> Description of Artificial Sequence: an artificially synthesized
primer sequence

<400> 49
ttggtacctg acctcaggta atccacct 28


<210> 50
<211> 29
<212> DNA
<213> Artificial Sequence

<220>
<223> Description of Artificial Sequence: an artificially synthesized
primer sequence

<400> 50
aaggtaccag tcttgtcgcc caggctgga 29


<210> 51
<211> 29
<212> DNA
<213> Artificial Sequence

<220>
<223> Description of Artificial Sequence: an artificially synthesized
primer sequence

<400> 51
taggtaccat aaatcacatg taactgagg 29

<210> 52
<211> 30
<212> DNA
<213> Artificial Sequence

<220>
<223> Description of Artificial Sequence: an artificially synthesized
primer sequence

<400> 52
agctagccca aactataaat atttagcatt 30

<210> 53
<211> 30
<212> DNA
<213> Artificial Sequence

<220>
<223> Description of Artificial Sequence: an artificially synthesized
primer sequence

<400> 53
aaggtaccaa caaataatta tgtggactca 30

<210> 54
<211> 21
<212> DNA
<213> Mus musculus

<400> 54
gtcttggcct caggcctctg g 21

<210> 55
<211> 24
<212> DNA
<213> Mus musculus

<400> 55
tacctcagtt acatgtgatt tatg 24

<210>  56
<211>  36
<212>  DNA
<213>  Artificial Sequence

<220>
<223>  Description of Artificial Sequence: an artificially synthesized
       primer sequence

<400>  56
agtctagaat ggggaccgcc aggctacgca agctgc                                36


<210>  57
<211>  28
<212>  DNA
<213>  Mus musculus

<400>  57
aggagatagg aacacccttg ggggtact                                         28


<210>  58
<211>  1412
<212>  DNA
<213>  Mus musculus

<220>
<221>  CDS
<222>  (1)..(1374)
<223>


<220>
<221>  mat_peptide
<222>  (73)..(1371)
<223>


<400>  58
atg ggg acc gcc agg cta cgc aag ctg caa ctg ctg ctt ctg ctg ggc        48
Met Gly Thr Ala Arg Leu Arg Lys Leu Gln Leu Leu Leu Leu Leu Gly
                -20                 -15                 -10

gct tgg agg gcg ctc gga ggt gcc gcg cgt tgc cgc gtc acc cta gtt        96
Ala Trp Arg Ala Leu Gly Gly Ala Ala Arg Cys Arg Val Thr Leu Val
            -5                  -1  1               5

ttg tcc ccg cag aag gca act agc gcc gtc tgc agg agc tca gag gcc 144
Leu Ser Pro Gln Lys Ala Thr Ser Ala Val Cys Arg Ser Ser Glu Ala
10 15 20

acc caa gac agc gaa ctg gcc acg ctg cgc atg cgc ctg ggt cgc cac 192
Thr Gln Asp Ser Glu Leu Ala Thr Leu Arg Met Arg Leu Gly Arg His
25 30 35 40

gag gag ctg ctg cgc gcg ctg caa agg cgt gcg gcg gag ggt ggt gcg 240
Glu Glu Leu Leu Arg Ala Leu Gln Arg Arg Ala Ala Glu Gly Gly Ala
45 50 55

ctc gcg gac gag gtg cgc gca ctg cgc gag cac agt ctc acc ctg aac 288
Leu Ala Asp Glu Val Arg Ala Leu Arg Glu His Ser Leu Thr Leu Asn
60 65 70

acg cgc ctg ggc cag ctg cgc gcg caa ttg cag cag gag gcg agg gcg 336
Thr Arg Leu Gly Gln Leu Arg Ala Gln Leu Gln Gln Glu Ala Arg Ala
75 80 85

gag cct gac ctg ggg gcg gag cct gct gct gca ctt ggt ttg cta gcc 384
Glu Pro Asp Leu Gly Ala Glu Pro Ala Ala Ala Leu Gly Leu Leu Ala
90 95 100

gag cgc gcg ctg gac gct gag gcc gaa gcg cgc cgg acg acg gca cgc 432
Glu Arg Ala Leu Asp Ala Glu Ala Glu Ala Arg Arg Thr Thr Ala Arg
105 110 115 120

ctg cag cag ctg gac gca cag ctc cgt gag cat gcg cag ctc atg agc 480
Leu Gln Gln Leu Asp Ala Gln Leu Arg Glu His Ala Gln Leu Met Ser
125 130 135

cag cat agc agc ctc ctc ggc cgc ctg caa cgc gcg tgc gcg ggc ccg 528
Gln His Ser Ser Leu Leu Gly Arg Leu Gln Arg Ala Cys Ala Gly Pro
140 145 150

gaa cgg gga cag cag cag gtc ctg cca ctg ccc ctg gcg cct ctg gtg 576
Glu Arg Gly Gln Gln Gln Val Leu Pro Leu Pro Leu Ala Pro Leu Val
155 160 165

cct ctg agc ctc gtg ggc agt gcc agc aac acc agc agg agg ctg gac 624
Pro Leu Ser Leu Val Gly Ser Ala Ser Asn Thr Ser Arg Arg Leu Asp
170 175 180

caa act cca gag cac cag aga gag cag agc ttg aga cag cag ggg cct 672
Gln Thr Pro Glu His Gln Arg Glu Gln Ser Leu Arg Gln Gln Gly Pro
185 190 195 200

cca tct tct ctg ctg ccc aca ggg cac ctt gct gtc ccc aca agg cca 720
Pro Ser Ser Leu Leu Pro Thr Gly His Leu Ala Val Pro Thr Arg Pro
205 210 215

gtg ggc cca tgg agg gat tgt gca gag gct cac ggg gca ggt cac tgg 768
Val Gly Pro Trp Arg Asp Cys Ala Glu Ala His Gly Ala Gly His Trp
220 225 230

cag agt gga gtg tat gac ctg cgg ctg ggc cgt cgt gta gta gcc gtg 816
Gln Ser Gly Val Tyr Asp Leu Arg Leu Gly Arg Arg Val Val Ala Val
235 240 245

tgg tgt gaa cag cag cag gaa ggt gga ggc tgg act gtc atc cag aga 864
Trp Cys Glu Gln Gln Gln Glu Gly Gly Gly Trp Thr Val Ile Gln Arg
250 255 260

cgg cag gac ggc tct gtc aac ttc ttc acc aac tgg cag cac tac aag 912
Arg Gln Asp Gly Ser Val Asn Phe Phe Thr Asn Trp Gln His Tyr Lys
265 270 275 280

gcg ggc ttt ggg cgt cca gaa gga gaa tac tgg ctg ggc ctg gaa cct 960
Ala Gly Phe Gly Arg Pro Glu Gly Glu Tyr Trp Leu Gly Leu Glu Pro
285 290 295

gtg cat cag gtg aca agc cgt ggg gac cac gag ctg ctg ata ctc cta 1008
Val His Gln Val Thr Ser Arg Gly Asp His Glu Leu Leu Ile Leu Leu
300 305 310

gag gac tgg ggg ggc cgt gca gca cgc gcc cac tac gac agc ttc tcc 1056
Glu Asp Trp Gly Gly Arg Ala Ala Arg Ala His Tyr Asp Ser Phe Ser
315 320 325

ttg gag cct gag agt gac cac tac cgt ctg cgg ctt ggc cag tac cac 1104
Leu Glu Pro Glu Ser Asp His Tyr Arg Leu Arg Leu Gly Gln Tyr His
330 335 340

ggc gat gcc gga gac tcc ctc tct tgg cac aat gac aaa cct ttc agc 1152
Gly Asp Ala Gly Asp Ser Leu Ser Trp His Asn Asp Lys Pro Phe Ser
345 350 355 360

act gtg gat agg gac aga gac tca tat tct ggt aac tgt gcc ctg tac 1200
Thr Val Asp Arg Asp Arg Asp Ser Tyr Ser Gly Asn Cys Ala Leu Tyr
365 370 375

cat cgt ggg ggc tgg tgg tac cat gcc tgt gcc cac tct aac ctc aat 1248
His Arg Gly Gly Trp Trp Tyr His Ala Cys Ala His Ser Asn Leu Asn
380 385 390

gga gta tgg tat cat gga ggt cat tac cgg agc cga tac cag gac ggg     1296
Gly Val Trp Tyr His Gly Gly His Tyr Arg Ser Arg Tyr Gln Asp Gly
        395                 400                 405

gtc tac tgg gcc gag ttc cgt ggt ggg gcg tac tct ctg aag aaa gct     1344
Val Tyr Trp Ala Glu Phe Arg Gly Gly Ala Tyr Ser Leu Lys Lys Ala
    410                 415                 420

gtt atg ttg acc cgg ctt gtg cgc ttg tgactgtccc atcagtaccc           1391
Val Met Leu Thr Arg Leu Val Arg Leu
425                 430

ccaagggtgt tcctatctcc t                                             1412


<210>  59
<211>  457
<212>  PRT
<213>  Mus musculus

<400>  59

Met Gly Thr Ala Arg Leu Arg Lys Leu Gln Leu Leu Leu Leu Leu Gly
                -20                 -15                 -10

Ala Trp Arg Ala Leu Gly Gly Ala Ala Arg Cys Arg Val Thr Leu Val
            -5                  -1  1               5

Leu Ser Pro Gln Lys Ala Thr Ser Ala Val Cys Arg Ser Ser Glu Ala
    10                  15                  20

Thr Gln Asp Ser Glu Leu Ala Thr Leu Arg Met Arg Leu Gly Arg His
25                  30                  35                  40

Glu Glu Leu Leu Arg Ala Leu Gln Arg Arg Ala Ala Glu Gly Gly Ala
                45                  50                  55

Leu Ala Asp Glu Val Arg Ala Leu Arg Glu His Ser Leu Thr Leu Asn
            60                  65                  70

Thr Arg Leu Gly Gln Leu Arg Ala Gln Leu Gln Gln Glu Ala Arg Ala
        75                  80                  85

Glu Pro Asp Leu Gly Ala Glu Pro Ala Ala Ala Leu Gly Leu Leu Ala
90 95 100

Glu Arg Ala Leu Asp Ala Glu Ala Glu Ala Arg Arg Thr Thr Ala Arg
105 110 115 120

Leu Gln Gln Leu Asp Ala Gln Leu Arg Glu His Ala Gln Leu Met Ser
125 130 135

Gln His Ser Ser Leu Leu Gly Arg Leu Gln Arg Ala Cys Ala Gly Pro
140 145 150

Glu Arg Gly Gln Gln Gln Val Leu Pro Leu Pro Leu Ala Pro Leu Val
155 160 165

Pro Leu Ser Leu Val Gly Ser Ala Ser Asn Thr Ser Arg Arg Leu Asp
170 175 180

Gln Thr Pro Glu His Gln Arg Glu Gln Ser Leu Arg Gln Gln Gly Pro
185 190 195 200

Pro Ser Ser Leu Leu Pro Thr Gly His Leu Ala Val Pro Thr Arg Pro
205 210 215

Val Gly Pro Trp Arg Asp Cys Ala Glu Ala His Gly Ala Gly His Trp
220 225 230

Gln Ser Gly Val Tyr Asp Leu Arg Leu Gly Arg Arg Val Val Ala Val
235 240 245

Trp Cys Glu Gln Gln Gln Glu Gly Gly Gly Trp Thr Val Ile Gln Arg
250 255 260

Arg Gln Asp Gly Ser Val Asn Phe Phe Thr Asn Trp Gln His Tyr Lys
265 270 275 280

Ala Gly Phe Gly Arg Pro Glu Gly Glu Tyr Trp Leu Gly Leu Glu Pro
                285                 290                 295

Val His Gln Val Thr Ser Arg Gly Asp His Glu Leu Leu Ile Leu Leu
            300                 305                 310

Glu Asp Trp Gly Gly Arg Ala Ala Arg Ala His Tyr Asp Ser Phe Ser
        315                 320                 325

Leu Glu Pro Glu Ser Asp His Tyr Arg Leu Arg Leu Gly Gln Tyr His
    330                 335                 340

Gly Asp Ala Gly Asp Ser Leu Ser Trp His Asn Asp Lys Pro Phe Ser
345                 350                 355                 360

Thr Val Asp Arg Asp Arg Asp Ser Tyr Ser Gly Asn Cys Ala Leu Tyr
                365                 370                 375

His Arg Gly Gly Trp Trp Tyr His Ala Cys Ala His Ser Asn Leu Asn
            380                 385                 390

Gly Val Trp Tyr His Gly Gly His Tyr Arg Ser Arg Tyr Gln Asp Gly
        395                 400                 405

Val Tyr Trp Ala Glu Phe Arg Gly Gly Ala Tyr Ser Leu Lys Lys Ala
    410                 415                 420

Val Met Leu Thr Arg Leu Val Arg Leu
425                 430